(19) Europäisches Patentamt
European Patent Office
Office européen des brevets

(11) **EP 2 149 582 A1**

## (12) EUROPEAN PATENT APPLICATION
published in accordance with Art. 153(4) EPC

(43) Date of publication:
**03.02.2010 Bulletin 2010/05**

(21) Application number: **08715312.8**

(22) Date of filing: **25.03.2008**

(51) Int Cl.:
*C07K 5/113* (2006.01)   *C07K 16/18* (2006.01)
*C07K 16/46* (2006.01)   *C12N 15/13* (2006.01)
*C12N 15/63* (2006.01)   *A61K 39/395* (2006.01)
*A61P 35/00* (2006.01)   *G01N 33/577* (2006.01)

(86) International application number:
**PCT/CN2008/070576**

(87) International publication number:
**WO 2008/128455 (30.10.2008 Gazette 2008/44)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **24.04.2007   CN 200710039873**

(71) Applicant: **Shanghai National Engineering Research Center of Antibody Medicine Co., Ltd. Zhangjiang Hi-Tech Park Shanghai 201-203 (CN)**

(72) Inventors:
• **GUO, Yajun**
 **Shanghai 201203 (CN)**

• **DAI, Jianxin**
 **Shanghai 201203 (CN)**
• **WANG, Hao**
 **Shanghai 201203 (CN)**
• **KOU, Geng**
 **Shanghai 201203 (CN)**
• **HOU, Sheng**
 **Shanghai 201203 (CN)**
• **QIAN, Weizhu**
 **Shanghai 201203 (CN)**
• **PENG, Ling**
 **Shanghai 201203 (CN)**

(74) Representative: **Martin Santos, Victoria Sofia et al C/Explanada 8 4˚ 28040 Madrid (ES)**

(54) **OSTEOPONTIN FUNCTIONAL EPITOPES, MONOCLONAL ANTIBODIES AGAINST THE EPITOPES AND USES THEREOF**

(57)   Provided are functional epitopes of osteopontin (OPN), monoclonal antibodies that specifically bind to the epitopes, immunoconjugates comprising the monoclonal antibodies and use of the monoclonal antibodies or immunoconjugates for manufacturing a medicament for the treatment of tumor. Also provided are nucleotide sequences encoding the monoclonal antibodies and vectors and host cells comprising the sequences. The monoclonal antibodies or immunoconjugates can be used for detecting the OPN, blocking the promoting metastasis-signaling pathway mediated by OPN and preventing the development and metastasis of tumor, thereby inhibiting the tumor.

EP 2 149 582 A1

Sequence Listing

<110>  SHANGHAI CP GUOJIAN PHARMACEUTICAL CO.Ltd

<120>  OSTEOPONTIN FUNCTIONAL EPITOPES, MONOCLONAL ANTIBODY
AGAINST THE EPITOPES AND USES THEREOF

<130>  081704pcwo

<150>  200710039873.3
<151>  2007-04-24

<160>  26

<170>  PatentIn version 3.3

<210>  1
<211>  942
<212>  DNA
<213>  Homo sapiens

<400>  1
atgagaattg cagtgatttg cttttgcctc ctaggcatca cctgtgccat
accagttaaa      60

caggctgatt ctggaagttc tgaggaaaag cagctttaca acaaatacc
agatgctgtg      120

gccacatggc taaaccctga cccatctcag aagcagaatc tcctagcccc
acagaatgct      180

gtgtcctctg aagaaaccaa tgactttaaa caagagaccc ttccaagtaa
gtccaacgaa      240

agccatgacc acatggatga tatggatgat gaagatgatg atgaccatgt
ggacagccag      300

gactccattg actcgaacga ctctgatgat gtagatgaca ctgatgattc
tcaccagtct      360

gatgagtctc accattctga tgaatctgat gaactggtca ctgattttcc
cacggacctg      420

ccagcaaccg aagttttcac tccagttgtc cccacagtag acacatatga
tggccgaggt      480

gatagtgtgg tttatggact gaggtcaaaa tctaagaagt ttcgcagacc
tgacatccag      540

taccctgatg ctacagacga ggacatcacc tcacacatgg aaagcgagga
gttgaatggt      600

gcatacaagg ccatccccgt tgcccaggac ctgaacgcgc cttctgattg
ggacagccgt      660

**19**

```
gggaaggaca gttatgaaac gagtcagctg gatgaccaga gtgctgaaac
ccacagccac     720

aagcagtcca gattatataa gcggaaagcc aatgatgaga gcaatgagca
ttccgatgtg     780

attgatagtc aggaactttc caaagtcagc cgtgaattcc acagccatga
atttcacagc     840

catgaagata tgctggttgt agaccccaaa agtaaggaag aagataaaca
cctgaaattt     900

cgtatttctc atgaattaga tagtgcatct tctgaggtca at
942


<210>   2
<211>   885
<212>   DNA
<213>   Mus musculus

<400>   2
atgagattgg cagtgatttg cttttgcctg tttggcattg cctcctccct
cccggtgaaa     60

gtgactgatt ctggcagctc agaggagaag ctttacagcc tgcacccaga
tcctatagcc     120

acatggccgg tgcctgaccc atctcagaag cagaatctcc ttgcgccaca
gaatgctgtg     180

tcctctgaag aaaaggatga ctttaagcaa gaaactcttc caagcaattc
caatgaaagc     240

catgaccaca tggacgacga tgatgacgat gatgatgacg atggagacca
tgcagagagc     300

gaggattctg tggactcgga tgaatctgac gaatctcacc attcggatga
gtctgatgag     360

accgtcactg ctagtacaca agcagacact ttcactccaa tcgtccctac
agtcgatgtc     420

cccaacggcc gaggtgatag cttggcttat ggactgaggt caaagtctag
gagtttccag     480

gtttctgatg aacagtatcc tgatgccaca gatgaggacc tcacctctca
catgaagagc     540

ggtgagtcta aggagtccct cgatgtcatc cctgttgccc agcttctgag
catgccctct     600

gatcaggaca acaacggaaa gggcagccat gagtcaagtc agctggatga
```

```
accaagtctg      660

gaaacacaca gacttgagca ttccaaagag agccaggaga gtgccgatca
gtcggatgtg      720

atcgatagtc aagcaagttc caaagccagc ctggaacatc agagccacaa
gtttcacagc      780

cacaaggaca agctagtcct agaccctaag agtaaggaag atgataggta
tctgaaattc      840

cgaatttctc atgaattaga gagttcatct tctgaggtca actag
885


<210>   3
<211>   351
<212>   DNA
<213>   Artificial


<220>
<221>   misc_feature
<223>   nucleotide sequence of the heavy chain variable region
in 1A12

<400>   3
caggttcagc tggagcagtc tggacctgag ctggtaaagc ctggggcttc
agtgaagatg      60

tcctgcaagt cttctggata cacattcact acctatgtta tgcactgggt
gaagcagaag      120

cctgggcagg gccttgagtg gattggatat attaatcctt acaatgatgg
tagtaagtac      180

aatgagaagt tcaaaggcaa ggccacactg acttcagaca aatcctccaa
cacagcctac      240

atggagctca gcagcctgac ctctgaggac tctgcggtct attactgtgc
aagccactac      300

ggtggtagcc ctgcttactg gggccaaggg actctggtca ctgtctctgc g
351


<210>   4
<211>   117
<212>   PRT
<213>   Artificial


<220>
<221>   misc_feature
```

<223> amino acid sequence of the heavy chain variable region in 1A12

<400> 4

Gln Val Gln Leu Glu Gln Ser Gly Pro Glu Leu Val Lys Pro Gly Ala
1               5                   10                  15

Ser Val Lys Met Ser Cys Lys Ser Ser Gly Tyr Thr Phe Thr Thr Tyr
            20                  25                  30

Val Met His Trp Val Lys Gln Lys Pro Gly Gln Gly Leu Glu Trp Ile
            35                  40                  45

Gly Tyr Ile Asn Pro Tyr Asn Asp Gly Ser Lys Tyr Asn Glu Lys Phe
        50                  55                  60

Lys Gly Lys Ala Thr Leu Thr Ser Asp Lys Ser Ser Asn Thr Ala Tyr
65                  70                  75                  80

Met Glu Leu Ser Ser Leu Thr Ser Glu Asp Ser Ala Val Tyr Tyr Cys
                85                  90                  95

Ala Ser His Tyr Gly Gly Ser Pro Ala Tyr Trp Gly Gln Gly Thr Leu
                100                 105                 110

Val Thr Val Ser Ala
            115


<210>   5
<211>   339
<212>   DNA
<213>   Artificial


<220>
<221>   misc_feature
<223>   nucleotide sequence of the light chain variable region in 1A12

```
<400>  5
gatattttga tgacccagac tccactctcc ctgcctgtca gtcttggaga
tcaagcctcc      60

atctcttgca gatctagtca gagccttgta cacagtaatg gaaacaccta
tttacattgg     120

tacctgcaga agccaggcca gtctccaaag ctcctgatct acaaagtttc
caaccgattt     180

tctggggtcc agacaggtt cagtggcagt ggatcaggga cagatttcac
actcaagatc     240

agcagagtgg aggctgagga tctgggagtt tatttctgct ctcaaagtac
acatgttccg     300

tggacgttcg gtggaggcac caagctggaa ataaaacgt
339


<210>  6
<211>  113
<212>  PRT
<213>  Artificial


<220>
<221>  misc_feature
<223>  amino acid sequence of the light chain variable region
in 1A12

<400>  6

Asp Ile Leu Met Thr Gln Thr Pro Leu Ser Leu Pro Val Ser Leu
Gly
1               5                   10                  15


Asp Gln Ala Ser Ile Ser Cys Arg Ser Ser Gln Ser Leu Val His
Ser
            20                  25                  30


Asn Gly Asn Thr Tyr Leu His Trp Tyr Leu Gln Lys Pro Gly Gln
Ser
        35                  40                  45


Pro Lys Leu Leu Ile Tyr Lys Val Ser Asn Arg Phe Ser Gly Val
Pro
    50                  55                  60
```

```
Asp Arg Phe Ser Gly Ser Gly Ser Gly Thr Asp Phe Thr Leu Lys
Ile
65              70              75                      80


Ser Arg Val Glu Ala Glu Asp Leu Gly Val Tyr Phe Cys Ser Gln
Ser
            85              90              95


Thr His Val Pro Trp Thr Phe Gly Gly Gly Thr Lys Leu Glu Ile
Lys
            100             105             110


Arg



<210>   7
<211>   314
<212>   PRT
<213>   Homo sapiens

<400>   7

Met Arg Ile Ala Val Ile Cys Phe Cys Leu Leu Gly Ile Thr Cys
Ala
1               5               10                      15


Ile Pro Val Lys Gln Ala Asp Ser Gly Ser Ser Glu Glu Lys Gln
Leu
            20              25              30


Tyr Asn Lys Tyr Pro Asp Ala Val Ala Thr Trp Leu Asn Pro Asp
Pro
            35              40              45


Ser Gln Lys Gln Asn Leu Leu Ala Pro Gln Asn Ala Val Ser Ser
Glu
    50              55              60


Glu Thr Asn Asp Phe Lys Gln Glu Thr Leu Pro Ser Lys Ser Asn
Glu
65              70              75                      80


Ser His Asp His Met Asp Asp Met Asp Asp Glu Asp Asp Asp Asp
His
            85              90              95
```

```
Val Asp Ser Gln Asp Ser Ile Asp Ser Asn Asp Ser Asp Asp Val
Asp
            100                 105                 110

Asp Thr Asp Asp Ser His Gln Ser Asp Glu Ser His His Ser Asp
Glu
            115                 120                 125

Ser Asp Glu Leu Val Thr Asp Phe Pro Thr Asp Leu Pro Ala Thr
Glu
            130                 135                 140

Val Phe Thr Pro Val Val Pro Thr Val Asp Thr Tyr Asp Gly Arg
Gly
145                 150                 155

160

Asp Ser Val Val Tyr Gly Leu Arg Ser Lys Ser Lys Lys Phe Arg
Arg
                  165                 170                 175

Pro Asp Ile Gln Tyr Pro Asp Ala Thr Asp Glu Asp Ile Thr Ser
His
            180                 185                 190

Met Glu Ser Glu Glu Leu Asn Gly Ala Tyr Lys Ala Ile Pro Val
Ala
            195                 200                 205

Gln Asp Leu Asn Ala Pro Ser Asp Trp Asp Ser Arg Gly Lys Asp
Ser
            210                 215                 220

Tyr Glu Thr Ser Gln Leu Asp Asp Gln Ser Ala Glu Thr His Ser
His
225                 230                 235

240

Lys Gln Ser Arg Leu Tyr Lys Arg Lys Ala Asn Asp Glu Ser Asn
Glu
                  245                 250                 255
```

```
His Ser Asp Val Ile Asp Ser Gln Glu Leu Ser Lys Val Ser Arg
Glu
         260              265              270


Phe His Ser His Glu Phe His Ser His Glu Asp Met Leu Val Val
Asp
         275              280              285


Pro Lys Ser Lys Glu Glu Asp Lys His Leu Lys Phe Arg Ile Ser
His
    290              295              300


Glu Leu Asp Ser Ala Ser Ser Glu Val Asn
305              310
```

```
<210>   8
<211>   32
<212>   DNA
<213>   Artificial


<220>
<221>   misc_feature
<223>   OPN sense primer

<400>   8
gggaagctta ccatgagaat tgcagtgatt tg
32


<210>   9
<211>   29
<212>   DNA
<213>   Artificial


<220>
<221>   misc_feature
<223>   OPN anti-sense primer

<400>   9
gccggtacca ttgacctcag aagatgcac
29


<210>   10
<211>   42
<212>   DNA
<213>   Artificial
```

```
<220>
<221>  misc_feature
<223>  mouse OPN sense primer

<400>  10
ataagcttgg atgacgacga caagatgaga attgcagtga tt
42


<210>  11
<211>  26
<212>  DNA
<213>  Artificial


<220>
<221>  misc_feature
<223>  mouse OPN anti-sense primer

<400>  11
atctcgagtt aattgacctc agaaga
26


<210>  12
<211>  18
<212>  DNA
<213>  Artificial


<220>
<221>  misc_feature
<223>  HGSP1 primer

<400>  12
gatactgtga tctgtttg
18


<210>  13
<211>  18
<212>  DNA
<213>  Artificial


<220>
<221>  misc_feature
<223>  HGSP2 primer

<400>  13
tcgcagatga gtctggac
18
```

```
<210>   14
<211>   18
<212>   DNA
<213>   Artificial


<220>
<221>   misc_feature
<223>   HGSP3 primer

<400>   14
atgaacacac tcacattg
18


<210>   15
<211>   24
<212>   DNA
<213>   Artificial


<220>
<221>   misc_feature
<223>   LGSP1 primer

<400>   15
gaggttatga ctttcatagt cagc
24


<210>   16
<211>   24
<212>   DNA
<213>   Artificial


<220>
<221>   misc_feature
<223>   LGSP2 primer

<400>   16
aacactgtcc aggacaccat ctcg
24


<210>   17
<211>   24
<212>   DNA
<213>   Artificial


<220>
<221>   misc_feature
```

<223> LGSP3 primer

<400> 17
tctgggatag aagttgttca tgag
24

<210> 18
<211> 351
<212> DNA
<213> Artificial

<220>
<221> misc_feature
<223> nucleotide sequence of the heavy chain variable region
(VH) in humanized anti-OPN antibody H1A12

<400> 18
caggtccagc ttgtccaaag tggagccgaa gtgaagaagc ccggagcaag
cgtgaaagtc     60

agctgtaaaa gcagtgggta caccttcact acttatgtta tgcactgggt
gaagcaagct     120

cctggtcagg gtctcgagtg gatcggatac attaacccat acaacgacgg
aagtaagtac     180

aatgagaagt ttaagggccg ggtgacaatc acaagtgaca agagtgcaag
caccgcctac     240

atggagctca gctccctgcg ttctgaagac accgccgtat attattgcgc
cagccattac     300

ggcggtagcc ctgcctactg gggtcagggt actctggtga cagtgtctag t
351

<210> 19
<211> 117
<212> PRT
<213> Artificial

<220>
<221> misc_feature
<223> amino acid sequence of the heavy chain variable region
(VH) in humanized anti-OPN antibody H1A12

<400> 19

Gln Val Gln Leu Val Gln Ser Gly Ala Glu Val Lys Lys Pro Gly
Ala
1               5                   10                  15

```
Ser Val Lys Val Ser Cys Lys Ser Ser Gly Tyr Thr Phe Thr Thr
Tyr
        20                  25                  30


Val Met His Trp Val Lys Gln Ala Pro Gly Gln Gly Leu Glu Trp
Ile
        35                  40                  45


Gly Tyr Ile Asn Pro Tyr Asn Asp Gly Ser Lys Tyr Asn Glu Lys
Phe
    50                  55                  60


Lys Gly Arg Val Thr Ile Thr Ser Asp Lys Ser Ala Ser Thr Ala
Tyr
65                  70                  75                  80


Met Glu Leu Ser Ser Leu Arg Ser Glu Asp Thr Ala Val Tyr Tyr
Cys
                85                  90                  95


Ala Ser His Tyr Gly Gly Ser Pro Ala Tyr Trp Gly Gln Gly Thr
Leu
            100                 105                 110


Val Thr Val Ser Ser
        115
```

```
<210>   20
<211>   339
<212>   DNA
<213>   Artificial


<220>
<221>   misc_feature
<223>   nucleotide sequence of the light chain variable region
(VL) in humanized anti-OPN antibody H1A12

<400>   20
gacgtcttga tgacccagtc ccccctttca ctcccagtaa cactaggcca
accggcctcg      60

atcagttgcc gcagctctca gtccctggtg cactcaaacg gaaatacgta
cttacattgg      120
```

```
ttccaacagc gacctgggca atcgcccaag ttgcttatat ataaagttag
taaccggttt     180

agcggtgtcc cagatcgttt ctctggctcc ggatcaggga ccgactttac
tctcaagatt     240

tcgcgcgtag aggcagaaga tgtgggtgtc tactattgta gtcagagcac
acacgtgccg     300

tggaccttcg gcggagggac taaagttgag atcaagcgg
339


<210>   21
<211>   113
<212>   PRT
<213>   Artificial


<220>
<221>   misc_feature
<223>   amino acid sequence of the light chain variable region
(VL) in humanized anti-OPN antibody H1A12

<400>   21

Asp Val Leu Met Thr Gln Ser Pro Leu Ser Leu Pro Val Thr Leu
Gly
1                   5                   10                  15


Gln Pro Ala Ser Ile Ser Cys Arg Ser Ser Gln Ser Leu Val His
Ser
                20                  25                  30


Asn Gly Asn Thr Tyr Leu His Trp Phe Gln Gln Arg Pro Gly Gln
Ser
            35                  40                  45


Pro Lys Leu Leu Ile Tyr Lys Val Ser Asn Arg Phe Ser Gly Val
Pro
        50                  55                  60


Asp Arg Phe Ser Gly Ser Gly Ser Gly Thr Asp Phe Thr Leu Lys
Ile
65                  70                  75                  80


Ser Arg Val Glu Ala Glu Asp Val Gly Val Tyr Tyr Cys Ser Gln
Ser
                85                  90                  95
```

Thr His Val Pro Trp Thr Phe Gly Gly Gly Thr Lys Val Glu Ile
Lys
                100                 105                110

Arg


<210> 22
<211> 990
<212> DNA
<213> Artificial


<220>
<221> misc_feature
<223> nucleotide sequence of the heavy chain constant region
(CH) in human antibody

<400> 22
gctagcacca agggcccatc ggtcttcccc ctggcaccct cctccaagag
cacctctggg     60

ggcacagcgg ccctgggctg cctggtcaag gactacttcc ccgaaccggt
gacggtgtcg    120

tggaactcag gcgccctgac cagcggcgtg cacaccttcc cggctgtcct
acagtcctca    180

ggactctact ccctcagcag cgtggtgacc gtgccctcca gcagcttggg
cacccagacc    240

tacatctgca acgtgaatca caagcccagc aacaccaagg tggacaagaa
agttgagccc    300

aaatcttgtg acaaaactca cacatgccca ccgtgcccag cacctgaact
cctgggggga    360

ccgtcagtct tcctcttccc cccaaaaccc aaggacaccc tcatgatctc
ccggacccct    420

gaggtcacat gcgtggtggt ggacgtgagc cacgaagacc ctgaggtcaa
gttcaactgg    480

tacgtggacg gcgtggaggt gcataatgcc aagacaaagc cgcgggaaga
gcagtacaac    540

agcacgtacc gtgtggtcag cgtcctcacc gtcctgcacc aggactggct
gaatggcaag    600

gagtacaagt gcaaggtctc caacaaagcc ctcccagccc ccatcgagaa

```
aaccatctcc      660

aaagccaaag ggcagccccg agaaccacag gtgtacaccc tgcccccatc
ccgggatgag      720

ctgaccaaga accaggtcag cctgacctgc ctggtcaaag gcttctatcc
cagcgacatc      780

gccgtggagt gggagagcaa tgggcagccg gagaacaact acaagaccac
gcctcccgtg      840

ctggactccg acggctcctt cttcctctac agcaagctca ccgtggacaa
gagcaggtgg      900

cagcagggga acgtcttctc atgctccgtg atgcatgagg ctctgcacaa
ccactacacg      960

cagaagagcc tctccctgtc tcccggtaaa
990
```

```
<210>   23
<211>   330
<212>   PRT
<213>   Artificial


<220>
<221>   misc_feature
<223>   amino acid sequence of the heavy chain constant region
(CH) in human antibody

<400>   23

Ala Ser Thr Lys Gly Pro Ser Val Phe Pro Leu Ala Pro Ser Ser
Lys
1               5                   10                  15


Ser Thr Ser Gly Gly Thr Ala Ala Leu Gly Cys Leu Val Lys Asp
Tyr
                20                  25                  30


Phe Pro Glu Pro Val Thr Val Ser Trp Asn Ser Gly Ala Leu Thr
Ser
                35                  40                  45


Gly Val His Thr Phe Pro Ala Val Leu Gln Ser Ser Gly Leu Tyr
Ser
        50                  55                  60
```

Leu Ser Ser Val Val Thr Val Pro Ser Ser Ser Leu Gly Thr Gln
Thr
65                    70              75                      80

Tyr Ile Cys Asn Val Asn His Lys Pro Ser Asn Thr Lys Val Asp
Lys
                 85              90                      95

Lys Val Glu Pro Lys Ser Cys Asp Lys Thr His Thr Cys Pro Pro
Cys
              100             105             110

Pro Ala Pro Glu Leu Leu Gly Gly Pro Ser Val Phe Leu Phe Pro
Pro
          115             120             125

Lys Pro Lys Asp Thr Leu Met Ile Ser Arg Thr Pro Glu Val Thr
Cys
      130             135             140

Val Val Val Asp Val Ser His Glu Asp Pro Glu Val Lys Phe Asn
Trp
145             150             155
160

Tyr Val Asp Gly Val Glu Val His Asn Ala Lys Thr Lys Pro Arg
Glu
              165             170             175

Glu Gln Tyr Asn Ser Thr Tyr Arg Val Val Ser Val Leu Thr Val
Leu
              180             185             190

His Gln Asp Trp Leu Asn Gly Lys Glu Tyr Lys Cys Lys Val Ser
Asn
      195             200             205

Lys Ala Leu Pro Ala Pro Ile Glu Lys Thr Ile Ser Lys Ala Lys
Gly
      210             215             220

Gln Pro Arg Glu Pro Gln Val Tyr Thr Leu Pro Pro Ser Arg Asp
Glu
225             230             235

240

Leu Thr Lys Asn Gln Val Ser Leu Thr Cys Leu Val Lys Gly Phe
Tyr
            245             250             255

Pro Ser Asp Ile Ala Val Glu Trp Glu Ser Asn Gly Gln Pro Glu
Asn
        260         265         270

Asn Tyr Lys Thr Thr Pro Pro Val Leu Asp Ser Asp Gly Ser Phe
Phe
        275         280         285

Leu Tyr Ser Lys Leu Thr Val Asp Lys Ser Arg Trp Gln Gln Gly
Asn
    290         295         300

Val Phe Ser Cys Ser Val Met His Glu Ala Leu His Asn His Tyr
Thr
305             310             315
320

Gln Lys Ser Leu Ser Leu Ser Pro Gly Lys
            325             330

<210> 24
<211> 318
<212> DNA
<213> Artificial

<220>
<221> misc_feature
<223> nucleotide sequence of the light chain constant region
(CL) in human antibody

<400> 24
actgtggctg caccatctgt cttcatcttc ccgccatctg atgagcagtt
gaaatctgga    60

actgcctctg ttgtgtgcct gctgaataac ttctatccca gagaggccaa
agtacagtgg    120

aaggtggata acgccctcca atcgggtaac tcccaggaga gtgtcacaga
gcaggacagc    180

35

```
aaggacagca cctacagcct cagcagcacc ctgacgctga gcaaagcaga
ctacgagaaa    240

cacaaagtct acgcctgcga agtcacccat cagggcctga gctcgcccgt
cacaaagagc    300

ttcaacaggg gagagtgt
318
```

```
<210>  25
<211>  106
<212>  PRT
<213>  Artificial

<220>
<221>  misc_feature
<223>  amino acid sequence of the light chain constant region
(CL) in human antibody

<400>  25
```

```
Thr Val Ala Ala Pro Ser Val Phe Ile Phe Pro Pro Ser Asp Glu
Gln
1                5                  10                  15


Leu Lys Ser Gly Thr Ala Ser Val Val Cys Leu Leu Asn Asn Phe
Tyr
            20                  25                  30


Pro Arg Glu Ala Lys Val Gln Trp Lys Val Asp Asn Ala Leu Gln
Ser
        35                  40                  45


Gly Asn Ser Gln Glu Ser Val Thr Glu Gln Asp Ser Lys Asp Ser
Thr
    50                  55                  60


Tyr Ser Leu Ser Ser Thr Leu Thr Leu Ser Lys Ala Asp Tyr Glu
Lys
65                  70                  75                  80


His Lys Val Tyr Ala Cys Glu Val Thr His Gln Gly Leu Ser Ser
Pro
                85                  90                  95


Val Thr Lys Ser Phe Asn Arg Gly Glu Cys
```

```
                      100                    105

<210>  26
<211>  66
<212>  DNA
<213>  Artificial


<220>
<221>  misc_feature
<223>  sequence of the signal peptide gene

<400>  26
atggattttc aggtgcagat tttcagcttc ctgctaatca gtgcctcagt
cataatatcc     60

agagga
66
```

## Description

### Technical field

The present invention relates to biotechnical field. In particular, the present invention discloses a functional protein epitope, a specific bound monoclonal antibody thereof, and the use thereof in the manufacture of antitumor agents.

### Technical Background

Tumor is one of the most important life-threatening diseases in our country. The post-surgery survival rate for five years is up to 40%, but there are still about half of the patients suffered from metastasis and recurrence after the surgery. How to control the high rate of tumor metastasis and recurrence after resection so as to enhance the therapeutic efficacy is of great important in international medical research. The intensive investigation of the mechanism of tumor cell metastasis will help clarifying the molecular mechanism of tumor metastasis and recurrence, understanding the signaling pathway of metastasis promoting, finding the effective target of metastasis inhibiting, providing a more effective target of blocking for drug development and clinical treatment, as well as improving the survival rate of cancer patients.

The research of molecular mechanism of tumor metastasis suggests that there are a variety of factors related with tumor cell transfer, such as p16 mutation, p53 mutation, p21, c-erbB-2, mdm-2, transforming growth factor $\alpha$ (TGF$\alpha$), epidermal growth factor receptor (EGF-R), matrix metalloproteinase -2 (MMP-2), urokinase-type plasminogen activator (uPA) and its receptor and plasminogen activator inhibitor-1 (PAI-1), cell intercellular adhesion molecule -1 (ICAM-1), vascular endothelial growth factor (VEGF), platelet-derived endothelial growth factor (PD-ECGF) and so on, which are invasive liver cancer positively correlated factors (Yamaguchi H, Wyckoff J, Condeelis J. Cell migration in tumors. Curr Opin Cell Biol. 2005; 17 (5):559-64. Huber MA, Kraut N, Beug H. Molecular requirements for epithelial-mesenchymal transition during tumor progression. Curr Opin Cell Biol. 2005; 17 (5): 548-58.).

Recent studies have shown that osteopontin (OPN) plays a critical role in tumor metastasis (Rangaswami H, Bulbule A, Kundu GC, Osteopontin; role in cell signaling and cancer progression. Trends Cell Biol. 2006; 16 (2): 79-87). With the efforts in researches across the world, there continues to be reported new findings about the OPN promoting signal pathway, which explain the function in promoting tumor metastasis from different perspectives. The important regulatory role of OPN signaling pathway in promoting tumor metastasis has become a hot-spot in the research of tumor metastasis.

Osteopontin is glycoprotein and an important pro-metastasis signal molecule, which is expressed in bone, kidney, brain, glandular epithelial cells, vascular smooth muscle cells, activated macrophages, lymphocytes and tumor cells (Weber GF, Ashkar S, Glimcher MJ, Cantor H. Receptor-ligand interaction between CD44 and osteopontin (Eta-1). Science (Washington, DC) 1996; 271: 509-12.), OPN may promote the extracellular matrix degradation by tumor cells, cell migration and cell apoptosis through activating the downstream receptor signaling pathway of CD44 (Miyauchi A, Alvarez J, Greenfield EM et al., Recognition of osteopontin and related peptides by an $\alpha$v$\beta$3 integrin stimulates immediate cell signals in osteoclasts, J Biol Chem 1991; 266:20369-7), and integrin proteins (Teramoto H, Castellone MD, Malek RL, Letwin N, Frank B, Gutkind JS, Lee NH. Autocrine activation of an osteopontin-CD44-Rac pathway enhances invasion and transformation by H-RasV12. Oncogene. 2005; 24 (3):489-501), both being surface receptors on tumor cells.

Specifically, OPN signal is recognized by receptor CD44, thereby inducing the activation of a small G protein of the Rho family in tumor cells, such as Rac (Teramoto H, et al., supra, Oncogene. 2005 Jan 13; 24 (3):489-501). The small G protein emits an extracellular chemotactic signal to a downstream effector protein such as a member of the WASP (Wiskott-Aldrich syndrome protein) family. The WASP protein binds and activates the actin-related protein (Arp2/3) complex, which catalyzes the actin polymerization reaction and induces the cytoskeleton reconstruction and the formation of cell membrane protrusion in tumor cells, whereby, the cell migration is enhanced (Wolf K, Mazo I, Leung H et al. Compensation mechanism in tumor cell migration: mesenchymal-amoeboid transition after blocking of pericellular proteolysis. J Cell Biol 2003; 160: 267-77). The activated WASP protein promotes the cell membrane protrusions to form an integrin-dependent cell adhesion on the stretched front edge of the migrating cells, and thereby induces a local accumulation of metalloproteinase in matrix and promotes the degradation of extracellular matrix (Nicholson, KM, and Anderson, NG The protein kinase B/Akt signaling pathway in human malignancy. Cell Signal. 2002; 14, 381-395). In addition, the OPN-CD44 downstream pathway can activates phosphatidylinositol 3-kinase (PI-3K), whose targets include Akt kinase. Akt kinase regulates the cell cycle, enhances the events including cell survival, cell anchorage-independent growth and cell migration, and mediates the OPN-promoted tumor anti-apoptosis and cell migration (Lin, YH, and Yang-Yen, HF The osteopontin-CD44 survival signaling involves activation of the phosphatidylinositol 3-kinase/Akt signaling pathway (J. Biol. Chem. 2001; 276, 46024-46030. Philip, S., and Kundu, GC Osteopontin induces nuclear factor $\kappa$B mediated promatrix metalloproteinase-2 activation through I kappa B alpha/IKK signaling pathways and curcumin (diferulolylmethane) downregulates these pathways. J. Biol. Chem. 2003; 278, 14487-14497). OPN, after being recognized

by the receptor $\alpha v\beta 3$, activates NIK and MEKK1, which induces the activation and nuclear localization of the downstream NF-$\kappa$B and AP-1 to up-regulate the expression of the effecter genes uPA and MMPs (Rangaswami, H. et al., (2004) Nuclear factor inducing kinase plays a crucial role in osteopontin induced MAPK / I$\kappa$Ba kinase dependent nuclear factor-$\kappa$B mediated promatrix metalloproteinase-9 activation, J. Biol. Chem. 279, 38921-38935. Rangaswami, H, et al., (2005) JNKI differentially regulates osteopontin induced nuclear factor inducing kinase/MEKK1 dependent activating protein-1 -mediated promatrix metalloproteinase-9 activation. J. Biol. Chem. 280, 19381-19392). At the same time, the OPN secreted by tumor cells can enhance the expression of the vascular endothelial growth factor VEGF through an autocrine pathway and/or a paracrine pathway, which in turn enhances the vascular endothelial cell proliferation and capillary formation in tumor (Goutam Chakraborty et al., (2008) Osteopontin Promotes Vascular Endothelial Growth Factor-Dependent Breast Tumor Growth and Angiogenesis via Autocrine and Paracrine Mechanisms. Cancer Res 2008; 68: 152-161). As said above, the OPN signaling pathway plays important regulatory roles in different aspects and different phases of invasion of the extracellular matrix by metastatic cells, diffusion into peripheral tissues or organs through blood or lymphatic vessels and formation of foci of metastasis.

It is expected that an anti-OPN antibody can block the OPN-mediated pro-metastasis signaling pathway, and thus effectively block tumorous adhesion and migration, prevent tumorous infiltration, inhibit tumorous capillary-formation, and finally prevents the development and metastasis of tumors.

## Description of the Invention

The purpose of the present invention is to provide a monoclonal antibody against specific epitopes of osteopontin, so as to treat tumor,

In the first aspect, the present invention provides a functional osteopontin epitope, which is expressed as NXPY, wherein X = A or G, Y = S, T, N, or P.

In a preferred embodiment, the functional epitope is NAPS. In another preferred embodiment, the functional epitope corresponds to amino acid residues 212-215 in exon 7 of osteopontin.

In the second aspect, the present invention provides an anti-osteopontin monoclonal antibody that specifically binds to the functional epitope.

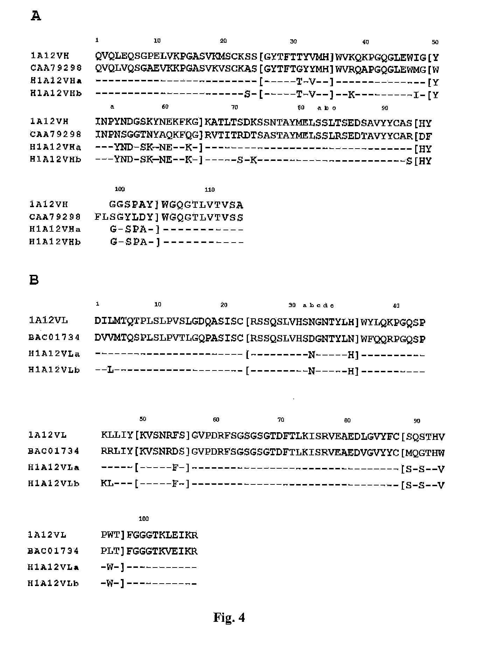

In a preferred embodiment, the CDRs in the variable region of the heavy chain (VH) of the monoclonal antibody each have an amino acid sequence selected from the group consisting of GYTFTTYVMH, YINPYNDGSKYNEKFKG or HYGGSPAY (for example, see H1A12VHb in Figure 4), and the CDRs in the variable region of the light chain (VL) each have an amino acid sequence selected from the group consisting of RSSQSLVHSNGNTYLH, KVSNRFS and SQSTHVPWT (for example, see H1A12VLb in Figure 4).

In another preferred embodiment, the VH region of the monoclonal antibody has an amino acid sequence as set forth in SEQ ID NO: 4 or SEQ ID NO: 19, and VL region has an amino acid sequence as set forth in SEQ ID NO: 6 or SEQ ID NO: 21.

In another preferred embodiment, the constant region of the monoclonal antibody is a mouse constant region or a human constant region.

In another preferred embodiment, the constant region is mouse IgG, In another preferred embodiment, the monoclonal antibody is obtained by a hybridoma or DNA recombination, or is isolated from a phage library of antibodies. In another preferred embodiment, the monoclonal antibody is a chimeric antibody or a humanized antibody.

In the third aspect, the present invention provides a DNA molecule, which encodes the monoclonal antibody of the invention.

In a preferred embodiment of the DNA molecule, the nucleotide sequence encoding the VH region is SEQ ID NO: 3 or SEQ ID NO: 18; and the nucleotide sequence encoding VL region is SEQ ID NO: 5 or SEQ ID NO: 20.

In the fourth aspect, the present invention provides a vector, which contains the DNA molecule of the invention mentioned above.

In the fifth aspect, the present invention provides a host cell, which contains the vector of the invention, or contains the DNA molecule of this invention integrated into the genome.

In a preferred embodiment, the host cell is a prokaryotic cell, preferably a bacterial cell; a lower eukaryotic cell, preferably a yeast cell; or a higher eukaryotic cell, preferably a mammalian cell.

In the sixth aspect, the present invention provides an immunoconjugate, which contains (a) the monoclonal antibody of the present invention and (b) a conjugating moiety selected from the group consisting of drugs, toxins, cytokines, radionuclides, and enzymes.

In the seventh aspect, the present invention provides uses of the anti-osteopontin monoclonal antibody and the immunoconjugate of the invention in the manufacture of antitumor agents.

In a preferred embodiment, the tumor is selected from the group consisting of adenocarcinoma, leukemia, lymphoma, melanoma, sarcoma; the tumor tissue selected from adrenal gland, gall bladder, bone, bone marrow, brain, breast, bile duct, gastrointestinal tract, heart, kidney, liver, lung, muscle, ovary, pancreas, parathyroid, penis, prostate, skin, salivary gland, spleen, testis, thymus, thyroid, or uterine; central nervous system tumors; ocular tumors, endocrine gland tumors, neuro-endocrine system tumors, gastrointestinal tract tumors, pancreatic cancer, tumors of endocrine system, reproductive system tumors or head and neck tumors.

In a preferred embodiment, the central nervous system tumor is glioma polymorphy or astrocytoma. In another preferred embodiment, the ocular tumor is basal cell carcinoma, squamous cell carcinoma or melanoma.

In the eighth aspect, the present invention provides a pharmaceutical composition, which contains the anti-osteopontin monoclonal antibody or the immunoconjugate of the invention and a pharmaceutically acceptable carrier.

In a preferred embodiment, the pharmaceutical composition contains 0.00001 - 99.9 wt%, preferably 0.0001-90 wt%, more preferably 0.001-75 wt%, more preferably 0.01-50 wt% of the monoclonal antibody or the immunoconjugate.

In another preferred embodiment, the pharmaceutical composition further contains an additional anti-tumor agent which can be selected from the group consisting of TNF-$\alpha$, TGF-$\beta$, IFN-$\alpha$, angiostatin, endostatin, glyfosfin, hemoporphyrin, lycobetaine, Bruceine, etoposide, anhydro-dulcit, adriamycin, tamoxifen, 5-fluorouracil, norcantharidin, tegadifur, cucurbitacin, harringtonine, rubescensine B, irisquinone, polysaccharide-peptide, cytarabine, carboplatin, paclitaxel, lentinan, flutamide, ifosfamide, ubenimex, leuprorelin, doxifluridine, lobaplatin, CPT-11, letrozole or teniposide.

In the ninth aspect, the present invention provides a kit for detection of osteopontin, which contains the anti-osteopontin monoclonal antibody or the immunoconjugate of the invention.

In the tenth aspect, the present invention provides a method for detecting the presence or the content of osteopontin in a biological sample, which includes the following steps: (i) contacting the sample with the anti-osteopontin monoclonal antibody or the immunoconjugate of the invention; (ii) detecting the formation of an antigen - antibody complex, wherein the formation of the antigen - antibody complex suggests the presence of osteopontin in the sample, or quantifying the antigen - antibody complex to determine the content of osteopontin in the sample.

In a preferred embodiment, the sample can be optionally pre-treated, preferably by extraction, purification and/or enrichment.

The other aspects of the present invention will be obvious to one skilled in the art from the detailed description below.

## DESCRIPTION OF THE DRAWINGS

Figure 1: SDS-PAGE electrophoretogram of the purified human and mouse OPN expressed in eukaryotic cells, wherein M represents protein molecular weight markers.

Figure 2: Western blot of mouse anti-hOPN mAb 1A12.

Figure 3: The mimic molecular structure of the humanized antibody h1A12; FR residues are shown as dark gray belts, CDR residues are shown as light gray belts, the nine important residues in the mouse FR regions within a distance of 5Å from CDR are shown as black ball rod.

Figure 4: The alignment between the amino acid sequences of the heavy chain (Figure 4A) and the light chain (Figure 4B) of the humanized antibody h1A12 and relevant sequences. Therein, 1A12VH and 1A12VL represents the VH and VL regions of the mouse-originated monoclonal antibody 1A12, respectively. The VH of the human antibody CAA79298.1 and the VL of the human antibody BAC01734.1 are used to construct the FR regions of the heavy chain and light chain in the humanized antibody h1A12, respectively. h1A12VHa and h1A12VHb represents two different humanized VH regions, and h1A12VLa and h1A12VLb represents two different humanized VL regions. The dashes represent the amino acid residues identical to those at the corresponding positions in the human antibody CAA79298.1 or BAC01734.1. CDRs are parenthesized. The amino acid residues are numbered in the Kabat's way [E.A. Kabat, T.T. Wu, H.M. Perry, K.S. Gottesman, C. Foeller, Sequences of Proteins of Immunological Interest, 5th edition, United States Department of Health and Human Services, Bethesda, MD, 1991.].

Figure 5: The antigen-binding activity of humanized antibody 1A12.

Figure 6: Tumor cell adhesion blocking effects of the mouse anti-hOPN mAb 1A12, the chimeric antibody c1A12 and the humanized antibody h1A12,

Figure 7: The inhibition of the tumorous invasion in the basement membrane by the mouse anti-hOPN mAb 1A12, the chimeric antibody c1A12 and the humanized antibody h1A12,

Figure 8: Scratch wound healing assay: the mouse anti-hOPN mAb 1A12, the chimeric antibody c1A12 and the humanized antibody h1A12.

Figure 9: The inhibition of the colony formation of tumor cells on soft agar of the mouse anti-hOPN mAb 1A12, the chimeric antibody c1A12 and the humanized antibody h1A12.

Figure 10: The effect of the mouse anti-hOPN mAb 1A12, the chimeric antibody c1A12 and the humanized antibody h1A12 on HUVEC cell proliferation.

Figure 11: The effect of the mouse anti-hOPN mAb 1A12, the chimeric antibody c1A12 and the humanized antibody h1A12 on HUVECs capillary tube formation.

Figure 12: The inhibition of chicken embryo chorioallantoic membrane (CAM) angiogenesis by the mouse anti-hOPN mAb 1A12, the chimeric antibody c1A12 and the humanized antibody h1A12, wherein the columns from left to right each represent PBS, VEGF, OPN, OPN + irrelevant antibody, OPN +1A12, OPN + c1A12 and OPN + h1A12.

Figure 13: The effect of the mouse anti-hOPN mAb 1A12, the chimeric antibody c1A12 and the humanized antibody h1A12 on OPN-induced corneal angiogenesis.

Figure 14: The inhibition of tumor growth by the mouse anti-hOPN mAb 1A12 in mice.

Figure 15: The inhibition of breast cancer metastasis by the mouse anti-hOPN mAb 1A12 in mice.

Figure 16: The effect of mouse anti-hOPN mAb 1A12 on the blood vessel density in the breast tumor tissue in mice.

Figure 17: The comparison of output efficiency after three cycles of biopanning for anti-OPN mAb 1A12.

Figure 18: ELISA and Western blot of anti-OPN mAb 1A12-positive phages;

Figure 18A is the ELISA of 1A12-positive phages, and Figure 18B is the ELISA and Western of 1A12-positive phages.

Figure 19: Align X sequence analysis of the binding epitopes of the anti-OPN monoclonal antibody 1A12.

Figure 20: Comparison of the binding affinity to the anti-OPN monoclonal antibody 1A12 between different epitope sequences.

Figure 21: The location of the epitopes specifically recognized by the anti-OPN monoclonal antibody 1A12 in the OPN molecule.

## DESCRIPTION OF THE EMBODIMENTS

The inventors obtained monoclonal antibodies that are specific for the functional epitopes in the osteopontin, and further produced chimeric monoclonal antibodies and humanized antibodies together and determined their encoding sequences. The inventors proved that the monoclonal antibody of the invention can inhibit tumor migration, and thereby can be used in the treatment of tumor.

Specifically, the human and the mouse OPN genes are cloned, and are expressed as human and mouse OPN proteins in eukaryotic cells. A murine anti-human OPN monoclonal antibody named 1A12 is produced using the cell fusion - hybridoma technique, the gene of which is further cloned and sequenced.

The invention disclosed a method of preparation of an anti-osteopontin humanized antibody, which includes a computer-aided design of the humanized antibody h1A12's amino acid sequence. Genes of the h1A12's VH and VL regions are synthesized, recombined with human CH and CV genes via recombination and splicing, cloned into a expression vector effective in eukaryotes to construct expression vectors respectively encoding the humanized light chain and the humanized heavy chain, the CHO cells are co-transfect with the light chain's and the heavy chain's expression vectors using the liposome technique, and then selection, culturing and purification are conducted to obtain the product. Chimeric monoclonal antibody c1A12 can also be obtained in a similar way.

A series of experiments using breast cancer cell line MDA-MB-435s show that the murine anti-human OPN monoclonal antibody 1A12, the human-mouse chimeric antibody c1A12 and the humanized antibody h1A12 of the invention can inhibit tumor metastasis. Cell adherent assays show that the anti-hOPN antibodies can effectively block the binding of MDA-MB-435s cells to hOPN. Cell invasion assays show that the anti-hOPN antibodies can effectively block the invasion of MDA-MB-435s into the basement membrane in the presence of hOPN. The scratches experiments show that the anti-hOPN antibodies can effectively inhibit the reparation of scratches in cells. The clone-forming assays on soft agars show that the anti-hOPN antibodies can inhibit the clone formation of MDA-MB-435s cells on the soft agar in size. The irrelevant antibodies as control do not show these effects. It can be seen then the anti-hOPN antibodies of the invention can effectively inhibit the formation of foci of metastasis.

A series of experiments in human vascular endothelial cell (HUVEC) show that the murine anti-human OPN monoclonal antibody 1A12, the human-mouse chimeric antibody c1A12 and the humanized antibody h1A12 can inhibit tumorous angiogenesis. $^3$H incorporation assays show that the anti-hOPN antibodies can effectively inhibit endothelial cell proliferation. Vascular structure analyses in HUVEC show that the anti-hOPN antibodies can inhibit the angiogenesis of vascular endothelial cell *in vitro.* Chicken embryo chorioallantoic membrane (CAM) angiogenesis assays and rabbit corneal neovascularization assays confirmed that the anti-hOPN antibodies can inhibit capillary formation *in vivo.* The irrelevant antibodies as control do not shown such effects. All the above suggest that the anti-hOPN antibodies of the invention can effectively inhibit angiopoiesis in tumors.

At the same time, the inventors also established an animal model of primary breast cancer and breast-to-pulmo metastasis in mice using the breast cancer cell line MDA-MB-435s. The model is used to evaluate the effect of the anti-OPN monoclonal antibody 1A12 in inhibiting tumor development, tumor metastasis and tumor angiogenesis. The irrelevant antibodies as controls do not show such effects. The above suggest that the anti-hOPN antibody 1A12 can effectively inhibit tumor development, tumor angiogenesis and block tumor metastasis.

The invention has also identified the functional epitope in OPN that interacts with the anti-hOPN antibody 1A12 by phage display. The functional epitope is found to be NAPS, which shows a site of target in the OPN molecular. More specifically, monoclonal antibody epitope panning, phage ELISA and Western blot are conducted to identify the sequences, and the sequences are analyzed to give putative functional epitopes in OPN. A series of short peptides are synthesized from the clone with the strongest binding capacity identified via phage cloning and analysis of antibody binding capacity. The functional epitopes are identified by testing these short peptides binding to the specific antibodies

The monoclonal antibody of the invention and its preparation

As used herein, the term "monoclonal antibody (mAb)" refers to an antibody from a substantially homogeneous population. That is, all the antibodies in the population are substantially the same, except for some naturally occurring mutations. A monoclonal antibody is highly specific to a unique antigenic site. Moreover, unlike the conventional polyclonal antibody preparations, which usually include different antibodies specific for different determinants, a monoclonal antibody is specific for a unique determinant on an antigen. In addition to their specificity, the advantages of monoclonal antibody also include that they are produced by a hybridoma culture and will not be contaminated by other immunoglobulins. The modifier "monoclonal" indicates the characteristic of the antibody being from a homogeneous population, which should not be interpreted as being limited to antibodies produced by a specific method.

As used herein, the term "antibody" or "immunoglobulin" refers to a heterotetramer glycoprotein of about 150,000 Dalton with characteristic structural features, which consists of two identical light chains (L) and two identical heavy chains (H). Each light chain is linked to a heavy chain through a covalent disulfide bond, and different immunoglobulin isotypes have different numbers of disulfide bonds between the two heavy chains. The heavy chains and the light chains each also have regularly spaced intra-chain disulfide bonds. At one end, each heavy chain has a variable region (VH) followed by multiple constant regions. Each light chain has a variable region (VL) at one end, and a constant region at the other. The light chain constant region corresponds to the first heavy chain constant region, and the VL region corresponds to the VH region. Specific amino acid residues form an interface between the light chain and the VH region.

As used herein, the term "variable" means that the variable regions of antibodies have domains of different sequences, which contributes to the specificity between antibodies and antigens. However, the variability is not evenly distributed throughout the variable regions. It is mainly located in the three domains in the VL and the VH region, which were called complementarity determining region (CDR) or hypervariable region. The more conservative part of the variable region is known as the framework region (FR). Natural heavy chain and light chain variable regions each contains four FRs, they generally in β-sheet configuration, connected by three CDR which formed connection loop, in some cases can form part of the b-folding structure. The CDR in each chain closely together through FR and form the antigen binding site with CDR of another chain (see Kabat et al., *NIH Publ.* No. 91-3242, Volume I, p647-669, (1991)). Constant region not directly involved in the binding of antibody and antigen, but showed different effects, such as involved in antibody-dependent cytotoxicity.

Vertebrate antibody (immunoglobulin) "light chain" may be classified as one of two significantly different classes (known as κ and λ) based on its constant region amino acid sequences. In accordance with its heavy chain constant region amino acid sequences, immunoglobulin can be divided into different types. There are five immunoglobulins: IgA, IgD, IgE, IgG and IgM, some of which can be further divided into subtypes (isotype), such as IgG1, IgG2, IgG3, IgG4, IgA, and IgA2. Corresponding to different types of immunoglobulin, the heavy chain constant region are known as α, δ, ε, γ, and μ. The subunit structure and three-dimensional conformation of different types of immunoglobulin is well known.

For example, the VH region of the monoclonal antibody of the invention may preferably have an amino acid sequence of SEQ ID NO: 4 or SEQ ID NO: 19, and the VL region may preferably have an amino acid sequence of SEQ ID NO: 6 or SEQ ID NO: 21, while the constant region can be from mouse or human, such as an IgG from mouse.

In one embodiment of the present invention, the CDRs 1-3 in the VH region each have the amino acid sequence selected from GYTFTTYVMH, YINPYNDGSKYNEKFKG or HYGGSPAY(see Figure 4 and H1A12VHb); and the CDRs 1-3 in the VL each have the amino acid sequence selected from RSSQSLVHSNGNTYLH, KVSNRFS or SQSTHVPWT (see Figure 4 and H1A12VLb).

Monoclonal antibodies can be prepared using a variety of well known methods. For example, monoclonal antibodies can be prepared by hybridoma method (first proposed by Kohler et al., Nature, 256:495 (1975)), or recombinant DNA methods (U.S. Patent No. 4,816,567). Monoclonal antibodies can also be isolated from phage antibody library according to for example, Clackson et al., Nature, 352:624-628 (1991) and Marks et al., J. Mol. Biol., 222:581-597 (1991).

The monoclonal antibody of the present invention can also be a chimeric antibody or a humanized antibody. In the invention, unless otherwise specified, "1A12" represents the murine anti-hOPN mAb 1A12, "c1A12" represents the chimeric anti-hOPN mAb c1A12 between human and murine, "h1A12" represents the humanized anti-hOPN mAb h1A12, In the c1A12, the VH region's amino acid sequence is SEQ ID NO: 4, and the VL's is SEQ ID NO: 6, the constant region is from human. In the h1A12, the VH region's amino acid sequence is SEQ ID NO: 19 and the VL's, is SEQ ID NO: 21, the constant region is from human.

The invention also includes the monoclonal antibodies that comprise an amino acid sequence corresponding to the anti-hOPN monoclonal antibody of the invention or fragments thereof, the monoclonal antibodies that comprises the variable region of the said anti-hOPN monoclonal antibody, and other proteins, conjugates and fusion expression products comprising same.

Specifically, the present invention includes proteins, conjugates and fusion expression products, such as an immunoconjugate and a fusion expression product, that comprises a hypervariable region (i.e., complementary determining region, CDR) at least 90%, preferably at least 95% homologous to the hypervariable region according to the present invention. As known in the art, immunoconjugates and fusion expression products include the conjugates formed between a drug, toxin, cytokine, radionuclide, enzyme and/or other diagnostic or therapeutic molecule and the anti-hOPN monoclonal antibody or fragments thereof. The invention also includes cell surface markers or antigens binding the anti-hOPN monoclonal antibody or fragments thereof.

The invention includes not only the intact monoclonal antibody, but also immuno-active antibody fragments, such as Fab or (Fab')$_2$, a heavy chain and a light chain.

Molecules encoding the anti-hOPN monoclonal antibodies or their fragments, expression vectors and host cells containing the molecules

The present invention also provides DNA molecules that encode the anti-hOPN monoclonal antibodies or fragments thereof. The sequence of these DNA molecules can be obtained by conventional techniques, such as PCR amplification or genome library screen. In addition, a sequence encoding the light chain can be fused to and a sequence encoding the heavy chain to form a single-chain antibody. For example, the DNA molecule of the present invention may comprise the nucleotide sequence of SEQ ID NO: 3 or SEQ ID NO: 18 encoding the VH and the nucleotide sequence of SEQ ID NO: 5 or SEQ ID NO: 20 encoding the VL. For example, c1A12 VH's encoding nucleotide sequence is shown in SEQ ID NO.3 and the VL's encoding nucleotide sequence in SEQ ID NO.5; h1A12 VH's encoding nucleotide sequence is shown in SEQ ID NO.18 and the VL's in SEQ ID NO.20. The invention also provided an expression vector containing the above nucleotide sequence of the present invention, which can be, for example, pcDNA3.1/ZEO (+) and pcDNA3.1 (+). The invention also provided host cells, such as COS or CHO cell, transformed with the vectors.

Production of a specific sequence can be scaled up using recombination techniques. Usually, the sequence is cloned into a vector to be transferred into the host cell, and finally isolated from the proliferated host cell culture.

The sequences can also be synthesized, especially for the short ones. Usually, short fragments are first synthesized, which are then connected into a long sequence.

The DNA sequence encoding the anti-hOPN monoclonal antibody or fragments or derivatives thereof according to the present invention can be obtained by whole-chemical synthesis. Then the DNA sequence can be introduced into a variety of existing DNA molecules (or vectors) and cells known in the art. Optionally, mutations can be introduced into the sequence of the invention by chemical synthesis.

The present invention also includes vectors that contain the above said DNA sequence and an appropriate promoter or other regulatory sequences. These vectors can be used to transform appropriate host cells to express the protein. The host cells can be prokaryotic cells, such as bacterial cell or lower eukaryotic cells, such as yeast cells or higher eukaryotic cells, such as mammalian cells.

The invention also provides a hybridoma cell line that can produce the anti-hOPN monoclonal antibodies of the invention. Preferably, the present invention provides a hybridoma cell line that can produce the anti-hOPN monoclonal antibodies at a high titer.

Upon obtaining the hybridoma, the skilled person can easily determine the structure of the anti-hOPN monoclonal antibody of the invention (such as the VH region and the VL region), and then prepare the monoclonal antibody, for example in the way as described below.

First, an expression vector which contains the nucleotide sequence of the monoclonal antibody of the invention is constructed.

As used herein, the term "expression-regulatory sequence" refers to a sequence contributes to the regulation of expression of nucleotide sequence. The expression-regulatory sequences include promoters and termination signals operably linked to target nucleotide sequences. They usually also include the sequences that are need for a proper translation of the nucleotide sequence. "Operably linked" means that certain parts of a linear DNA sequence can affect the activity of the other parts. For example, when a promoter or an enhancer increases the transcription of an encoding sequence, it is said to be operably linked to the encoding sequence.

The DNA sequences encoding the monoclonal antibody of the invention can be produced by any appropriate methods -known in the art. For example, the nucleotide sequences encoding the monoclonal antibody's VH region and VL region can be synthesized or amplified via PCR based on the herein disclosed sequences. Then restriction sites are selected as suitable to insert introduce the nucleotide sequences into an appropriate expression vector, so that they are ahead of sequence encoding the heavy chain's constant region (CH) encoding and sequence encoding the light chain's constant region (CL) respectively, and in the same reading frame with the encoding sequences. The expression vectors used in the present invention uses are commercially available, such as pPICZα, pPIC9K.

The expression vector constructed above is then used to transform the suitable host cell. The term "Host cell" includes prokaryotic cells and eukaryotic cells. Examples of commonly used prokaryotic host cells include *E. coli, Bacillus subtilis* and so on. Examples of commonly used eukaryotic host cells include yeast cells, insect cells, and mammalian cells. In the invention, mammalian cell is preferred. Mammalian cell line is usually used as host cell to express eukaryotic peptides. The culture and proliferation of mammalian cells are well known in the art. See "Tissue Culture", Academic Press, Kruse and Patterson Ed. (1973), the disclosures of which are hereby incorporated by reference. Preferred mammalian cells are immortalized cell lines, which can be commercially available. Such cell lines include but are not limited to the Chinese hamster ovary (CHO) cell, the Vero cell, the Hela cell, the baby hamster kidney (BHK) cell, the monkey kidney cell (such as COS), the human hepatocellular carcinoma cell (such as Hep G2). They provide post-translation modification of the protein, including correct folding, correct disulfide bond formation and correct glycosylation.

There are many methods to transfer host cell with expression vector, and the transformation procedure used depends on the host to be transformed. The method of introducing heterologous polynucleotide into mammalian cells is well known in the field, which includes dextran-mediated transfection, calcium phosphate precipitation, Polybrene-mediated transfection, protoplast fusion, electroporation, liposome-mediated transfection as well as direct DNA microinjection into the nucleus. In this invention, the preferred include electroporation and liposome-mediated transfection. For example, the Invitrogen liposome kit can be used to transfect host cells such as COS or CHO cells.

The transformed host cells are then cultured under conditions effective for the expression of the monoclonal antibody. Then the humanized anti-hOPN monoclonal antibody of the invention can be obtained in the same way as the conventional immunoglobulin purification, using well known separation and purification methods, such as protein A-Sepharose, hydroxyapatite chromatography, gel electrophoresis, dialysis, ion-exchange chromatography, hydrophobic chromatography, molecular sieve chromatography, and affinity chromatography, etc.

Identification, expression and purification of the monoclonal antibody

The resultant monoclonal antibody can be identified by conventional procedures. For example, the binding specificity of monoclonal antibody can be determined by immunoprecipitation or *in vitro* binding assay (such as radioimmunoassay (RIA) or enzyme-linked immunosorbent assay (ELISA)). The binding affinity of monoclonal antibody can be determined by, for example, the Scatchard analysis of Munson, et al., Anal. Biochem., 107:220 (1980).

The anti-hOPN monoclonal antibody of the invention can be expressed in or on the surface of the cell, or be secreted into the outside. The recombinant proteins can be separated and purified using a variety of separation methods, depending on its physical, chemical, and other features that need to be considered. These methods are well known in the art. Examples include, but not limited to, conventional refolding treatment, treatment with the protein precipitating agent (salting-out method), centrifugation, filtration, ultrasound treatment, ultracentrifugation, molecular sieve chromatography (gel filtration), adsorption chromatography, ion-exchange chromatography, high performance liquid chromatography (HPLC) and various other liquid chromatography techniques and their combinations.

Pharmaceutical compositions

The present invention also provides a pharmaceutical composition for the treatment of tumor, which contains a pharmaceutically effective amount of the monoclonal antibody of the invention or immunoconjugate thereof and a pharmaceutically acceptable carrier.

As used herein, the term "pharmaceutically acceptable" refers to when administering to animals or human the molecular itself and combinations will not result adverse, allergic or other adverse reactions. As used herein, the term "pharmaceutically acceptable carrier" should be compatible with the invention active agents, that is, can be combined and will not significant reduce the effect of the pharmaceutical composition under normal circumstances. These carriers are well known in the field. A thorough discussion of pharmaceutically acceptable carriers can be found in Remington's Pharmaceutical Sciences, Mack Pub. Co., NJ 1991.

Such carriers include, but not limited to, saline, buffers, glucose, water, glycerin, ethanol, adjuvants, and combinations thereof. In addition, these carriers may also exist in auxiliary substances, such as wetting agents or emulsifying agent, pH buffering substances and so on.

The composition of the invention can be administered orally intravenously, intramuscularly or subcutaneously, wherein oral or intravenous administration is preferred.

Typically, the present monoclonal antibody or immunoconjugate is contained in the present pharmaceutical composition at an amount of 0.00001 - 99.9 wt%; preferably 0.0001 - 90wt%, more preferably 0,001 - 75wt% and even more preferably 0.01 - 50wt%, based on the total weight of the composition. Balance are the pharmaceutical acceptable carrier and, optionally, other additives.

The pharmaceutical composition of the invention can be formulated as a variety of dosage forms as needed, and can be administered in an effective amount by a physician according to patient type, age, weight and general conditions, delivery routes and so on. Delivery routes can be, for example, infusion and others.

The pharmaceutical composition is used by administrating a safe and effective amount of the anti-hOPN monoclonal antibody or immunoconjugate to a mammalian subject, wherein the safe and effective amount is usually about 0.1-5 mg/kg body weight, in most cases, no more than about 5 mg/kg body weight, and is preferably in the range of about 1-10 μg/kg body weight to about 1 mg/kg body weight. Of course, for the specific dosage, administration routes and patient health status should also be taken into account, which is within the scope of skilled physicians.

The monoclonal antibody and immunoconjugate of the invention can inhibit tumor adhesion and migration, prevent tumor invasion, accelerate the apoptosis of tumor cells, and thus can be used to treat many kinds of tumors, which include but are not limited to adenocarcinoma, leukemia, lymphoma, melanoma, sarcoma; the tumor tissue from adrenal gland, gall bladder, bone, bone marrow, brain, breast, bile duct, gastrointestinal tract, heart, kidney, liver, lung, muscle, ovary, pancreas, parathyroid, penis, prostate, skin, salivary gland, spleen, testis, thymus, thyroid, or uterine; central nervous system tumors such as gliocytoma or astrocytoma; ocular tumors (such as basal cell carcinoma, squamous cell carcinoma or melanoma), endocrine tumors, neuro-endocrine system tumors, gastrointestinal tract-pancreas-endocrine system-tumors, reproductive system tumors or head and neck cancer.

The pharmaceutical composition of the present invention may also contain or be used in combination with an additional antitumor agent for additional benefits. The useful additional antitumor active agents include but are not limited to: TNF-α, TGF-β, IFN-α, angiostatin, endostatin, glyfosfin, hemoporphyrin, lycobetaine, Bruceine, etoposide, anhydrodulcit, adriamycin, tamoxifen, 5-fluorouracil, norcantharidin, tegadifur, cucurbitacin, harringtonine, rubescensine B, irisquinone, polysaccharide-peptide, cytarabine, carboplatin, paclitaxel, lentinan, flutamide, ifosfamide, ubenimex, leuprorelin, doxifluridine, lobaplatin, CPT-11, letrozole or teniposide and so on.

When two or more drugs are used in combination, they can usually provide a synergistic effect, which is much better than each being used alone. Preferably, the additional agents to be used in combination do not interfere desired activity of the monoclonal antibody of the invention.

hOPN Detection Kit

The invention also provides a hOPN detection kit, which contains the anti-hOPN monoclonal antibody or a fragment or an immunoconjugate thereof. The present kit can be used to detect the presence and/or to determine the content of osteopontin in a biological sample by a method including the following steps: (i) contacting the test sample with the anti-osteopontin monoclonal antibody or immunoconjugate thereof, and (ii) detecting the formation of an antigen - antibody complex, wherein the formation of the antigen - antibody complex suggests the presence of osteopontin in the sample. The formed antigen - antibody complex may or quantified to determine the content of osteopontin in the sample. The sample can optionally be pre-treated, for example, by extraction, purification, or enrichment.

The kit includes the present monoclonal antibody in containers or on plates and an instruction. The kit may also include additional reagents needed for the detection, such as buffers, indicators and so on. The skilled person can modify the contents of the kit as needed.

**Examples**

The invention will be further described in the following examples and tests, which should not be understood as to limit the scope of the invention to any particulars. Details of some traditional techniques, such as those used to construct vectors and plasmids, to insert protein encoding genes into these vectors and plasmids, to introduce plasmids into host cells, as well as the classic cell fusion and monoclonal antibody screening and purification methods are omitted, which have been well known in the art and can be easily find in many publications, including the Molecular Cloning: A Laboratory Manual, Sambrook, J., Fritsch, E.F. and Maniais, T. (1989),Cold spring Harbor Laboratory Press. All the percentages and parts are based on weight, unless otherwise indicated. All the materials used in the examples and tests are commercially available, unless otherwise specified.

**Example The preparation of anti-OPN monoclonal antibody**

Example 1. Cloning of the cDNA of Human OPN

The following primers were synthesized according to the sequence of human OPN form the GENEBANK:

OPN sense primer (primer 1);
GGG AAGCTT ACCATGAGAATTGCAGTGATTTG (Hind III) (SEQ ID NO: 8)
OPN antisense primer (primer 2);

GCC GGTACC ATTGACCTCAGAAGATGCAC (Kpn I) (SEQ ID NO: 9).

Human liver cancer cell line LM3 (purchased from Shanghai Zhongshan Hospital) was cultured and proliferated before extracting RNA by RT-PCR (PROMEGA) using TRISOL kit (INVITROGEN), The PCR condition was: 94˚C for 5 mins 30 cycles of 94˚C for 45 sec, 58˚C for 30 sec and 72˚C for 45 sec, and 72˚C for 10 mins. The target DNA fragment was 963bp. The fragment was recovered using a gel extraction kit (Shanghai Sangon), then digested with the restriction enzymes Hind III and Kpn I. The target fragment was recovered from the electrophoresis gel, and inserted into the plasmid vector digested with the Hind III and Kpn I. The recombinant vector was transformed into the strain of E. coli DH10B. The positive clones with the inserts were selected. Sequencing confirms the nucleotide sequence of the human OPN gene as set forth in SEQ ID NO: 1.

Example 2, Cloning of the cDNA of Murine OPN

The following primers were synthesized according to the sequence of murine OPN from the GENEBANK;

Murine OPN sense primer (primer 3);
AT AAGCTT GGATGACGACGACAAGATGAGAA TTGCAGTGATT (Hind III) (SEQ ID NO; 10)
Mouse OPN antisense primer (primer 4);
AT CTCGAG TTAATTGACCTCAGAAGA (Kpn I) (SEQ ID NO: 11),

The murine spleen T lymphocytes were isolated and activated for 30 hours with ConA. Total RNA was extracted using TRISOL kit (INVITROGEN). RT-PCR (PROMEGA) condition was: 94˚C for 5 mins, 30 cycles of 94˚C for 45 sec, 55˚C for 30 sec and 72˚C for 45 sec, and 72˚C for 10 mins. The target DNA fragment was 932bp in length. The fragment was recovered using a gel extraction kit (Shanghai Sangon), and digested with the restriction enzymes Hind III and Kpn I. The target fragment was recovered from the electrophoresis gel, and inserted into the plasmid vector digested with Hind III and Kpn I. The recombinant vector was transformed into the strain of E. coli DH10B. The positive clones with the inserts were selected. Sequencing confirms the nucleotide sequence of the murine OPN gene as set forth in SEQ ID NO: 2.

Example 3. Expression and purification of the human and the murine OPNs in eukaryotic cell

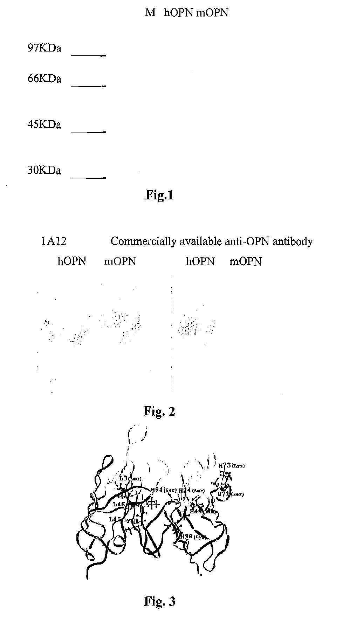

The correct inserts of the human and the murine OPN genes obtained in example 2 were recovered using appropriate restriction enzymes, and then inserted into the pPICZ$\alpha$ plasmid. The plasmids were used to transfect the strain of *Saccharomyces Pichia.* A single clone was selected, and the expression of the human and the murine OPN proteins was induced. The supernatant of yeast culture was collected, and purified with anion exchange and molecular sieve. SDS-PAGE showed that purified proteins of the human and the mouse OPNs were obtained. SDS-PAGE of the purified protein was shown in Figure 1.

Example 4. Screen and preparation of the murine anti-human OPN monoclonal antibody

100ug human OPN and an equal volume of Freund's adjuvant were emulsified, and injected intraperitoneally into BALB/C mice. Boost was given every two weeks at the same dose. After three immunizations, the mouse exhibiting a high serum anti-OPN antibody titer was selected, and spleen lymphocytes were isolated. The mouse spleen lymphocytes were fused with NS-1 cells using the classic PEG method. 96-well plate coated with 10$\mu$g/ml human OPN was used for repeated screening by ELISA method to obtain hybridoma cell line 1A12 which was capable of stably expressing anti-human OPN antibody. The strain of 1A12 was amplified, and administrated to BALB/C mice in $5 \times 10^6$ cells/mouse (i.p.). The ascites was collected from the $10^{th}$ day after the injection. The anti-human OPN monoclonal antibody was purified by affinity chromatography using protein A column.

The results of western blot showed that the mouse anti-human OPN monoclonal antibody 1A12 not only specifically bound to the human OPN protein, but also cross-reacted with the mouse OPN protein. The results were shown in Figure 2.

Example 5, Screen and preparation of the irrelevant murine anti-human OPN monoclonal antibody 23C3D3 as a control

100ug human OPN and an equal volume of Freund's adjuvant were emulsified, and administrated to BALB/C mouse (i.p.). The mice were boosted every two weeks with the same dose. After three immunizations, the mouse exhibiting a high serum anti-OPN antibody titer was selected, and spleen lymphocytes isolated. The mouse spleen lymphocytes were fused with NS-1 cells using the classic PEG method. A 96-well plate was coated with 10ug/ml KLH-WLVPDP (synthesized by Shanghai Yeli Co.) and used in repeated screening via ELISA to obtain the hybridoma cell line 23C3D3 which stably expresses the anti-human OPN antibody. The cell line was amplified and administrated to BALB/C mice in $5 \times 10^6$ cells/mouse (i.p.). The ascites was collected from the 10th day after the administration. Purification via affinity chromatography using protein A column gave an irrelevant monoclonal antibody against human OPN epitope, which was named 23C3D3. The antibody was taken as a control.

Example 6, Cloning and Sequencing of the genes of the 1A12 mAb's variable regions

$5 \times 10^6$ - $1 \times 10^7$ hybridoma cells 1A12 were collected. The total RNA was extracted using TRIzol (Invitrogen Catalog No. 15596-026), The following primers were designed according to the sequences of the mouse constant regions:

HGSP1: 5'-GATACTGTGATCTGTTTG-3' (SEQ ID NO: 12)
HGSP2: 5'-TCGCAGATGAGTCTGGAC-3' (SEQ ID NO: 13)
HGSP3: 5'-ATGAACACACTCACATTG-3' (SEQ ID NO: 14)
LGSP1; 5'-GAGGTTATGACTTTCATAGTCAGC-3' (SEQ ID NO: 15)
LGSP2: 5'-AACACTGTCCAGGACACCATCTCG-3' (SEQ ID NO: 16)
LGSP3: 5'-TCTGGGATAGAAGTTGTTCATGAG-3' (SEQ ID NO: 17)

Using Invitrogen 5'RACE kit (Catalog No. 18374-058), the first-strand cDNA was synthesized using the primers HGSP1 and LGSP1. The first-strand cDNA was treated with TdT and dCTP to add a poly C at the 3' end. HGSP2, HGSP3, LGSP2 and LGSP3 were used as 5' primers to prepare the VH and VL by nested-PCR. The PCR products were cloned into the pGEM-T vector. Plasmids were extracted from the selected clones. Positive clones was identified by restriction digestion and sequenced. The nucleotide sequence and the amino acid sequence of 1A12 mAb's VH were as set forth in SEQ ID NO: 3 and SEQ ID NO: 4 respectively, and the sequences of the VL were as set forth in SEQ ID NO: 5 and SEQ ID NO: 6 respectively.

Example 7. Cloning of the Genes of the Human antibody's constant regions

Healthy human lymphocytes were isolated using the lymphocyte separation medium (DingGuo Biotechnology Development Co.). The total RNA was extracted using Trizol reagent (Invitrogen). The primers were designed according to reported sequences in Cell, 1980, 22: 197-207 and Nucleic Acids Research, 1982, 10: 4071-4079, and used to amplify the genes of constant regions by RT-PCR. The PCR products were purified by agarose gel electrophoresis, and cloned into the pGEM-T vector. Correct insertion was verified by sequencing. SEQ ID NO: 22 and SEQ ID NO: 23 show the nucleotide sequence and the amino acid sequence of the heavy chain constant region (CH), and SEQ ID NO: 24 and SEQ ID NO: 25 show the nucleotide sequence and the amino acid sequence of the light chain constant region (CL). The clones with the correct inserts were named pGEM-T/CH and pGEM-T/CL.

Example 8. Construction of the chimeric anti-OPN antibody c1A12

The gene of chimeric antibody's heavy chain was synthesized by overlap PCR using the gene of the 1A12 VH region (1A12VH) and the pGEM-T/CH vector as the templates. The reaction condition was: 95˚C for 15 mins; 30 cycles of 94˚C for 50 sec, 58˚C for 50 sec, 72˚C for 50 sec; 72˚C for 10 mins. The gene of chimeric heavy chain was constructed to have a Hind IIIsite and a signal sequence at the 5' end and translation stop codon TAA and an EcoRI site at the 3' end. The signal sequence was ATGGATTTTCAGGTGCAGATTTTCAGCTTCCTGCTAATCA GTGCCTCAGTCATAATATCCAGAGGA (SEQ ID NO: 26). PCR products were separated on agarose gel, and the target band was recovered and cloned into the pGEMT vector. The positive clones were selected and sequenced. The clones containing the correct sequence were selected and digested with Hind III and EcoR I. The chimeric heavy chain c1A12VHCH was purified and recovered via electrophoresis on agarose gel, and inserted into to the plasmid pcDNA3.1 (+) (Invitrogen) double-digested with Hind III and EcoR I to construct pcDNA3,1 (+) (c1A12VHCH), the eukaryotic expression vector for the chimeric heavy chain.
The gene of the chimeric antibody's light chain was synthesized by overlap PCR using the 1A12 VL gene (1A12 VL) and the pGEM-T/CL vector as the templates. The reaction condition was: 95˚C for 15 mins; 30 cycles of 94˚C for 50 sec, 58˚C for 50 sec, 72˚C for 50 sec; 72˚C for 10 mins. Obtained was the PCR product has a Hind IIIsite and a signal sequence at the 5' end and the translation stop codon TAA and a EcoR I site at the 3' end. The signal peptide encoding sequence was ATGGATTTTCAGGTGCAGATTTTCAGCTTCCTGCTAATCAGTGC CTCAGTCATAATATCCAGAGGA (SEQ ID NO:26). PCR product was recovered by agarose gel electrophoresis and cloned into the pGEM-T vector. The positive clones were selected and sequenced. The clones containing the correct sequence were selected and digested with Hind III and EcoR I. The chimeric light chain c1A12VLCL was purified and recovered via electrophoresis on agarose gel, and inserted into the plasmid pcDNA3.1/ZEO(+) (Invitrogen) double-digested with Hind III and EcoR I to construct the vector pcDNA3.1/ZEO(+)(c1A12VLCL) eukaryotic expression vector for the chimeric light chain.

The constructed expression vectors for the chimeric heavy chain and the chimeric light chain were used to co-transfect the COS-1 cells (ATCC CRL 1650) using the liposome method. 72 hours later, the supernatant was collected, and the content of the antibody c1A12 therein was determined by ELISA. Specifically, an ELISA plate was coated with goat anti-human IgG (Fc) and blocked with 2% BSA-PBS at 37˚C for 2 hours. The supernatant to be tested and standard (Human myeloma IgG1, k) were added to the plate and incubated at 37˚C for 2 hours, HRP-goat anti-human κ was added to induce the binding reaction, and the plate was incubated at 37˚C for 1 hour. TMB was added and incubate at 37˚C for another 5 minutes. Finally, added $H_2SO_4$ was added to quench the reaction, and the value of OD450 was measured.

Example 9. Homologous modeling of the three-dimensional structure of murine 1A12's variable regions (Fv)

Insight II software package (Accelrys) was used to mimic the three-dimensional structure of 1A12 murine monoclonal antibody's variable region. First, the Protein Data Bank (PDB) was searched for the template proteins for the 1A12's VH and VL using BLAST program. Antibody 1PLG with the highest homology was selected as the template for the 1A12's model, and a model of the three-dimensional structure of 1A12 was constructed using Insight II program (Figure 3).

Example 10, Design and Construction of the Humanized Anti-Opn Antibody h1A12

BLAST program was used to search the Genebank database for the human-originated templates most similar to the 1A12's VL and VH regions respectively. The human-originated antibody the most homologous to 1A12's VH region (with a similarity of 67%) is the human antibody CAA79298.1 (emb|CAA79298.1|), and human-originated antibody the most homologous to 1A12's VL region (with a similarity of 81%) is BAC01734.1 (dbj|BAC01734.1|). Thus, CAA79298.1 and BAC01734.1 were used as the templates respectively for the 1A12's heavy chain and light chain.

First, the CDRs on the 1A12's heavy chain and light chain were directly transplanted to the human-originated template CAA79298.1 and BAC01734.1 respectively to construct the CDR-grafted antibodies, the heavy chain's was named h1A12Ha and light chain's, h1A12La, The sequences of the h1A12Ha's and the h1A12La's variable regions were shown in Figure 4. The genes of the VH and the VL of the humanized antibody (h1A12VHa and h1A12VLa) were obtained via whole gene synthesis. Then, the humanized antibody heavy chain's gene was synthesized by overlap PCR using the h1A12VHa gene and the pGEM-T/CH vector as templates, wherein the reaction condition was: 95˚C for 15 mins; 30 cycles of 94˚C for 50 sec, 58˚C for 50 sec, 72˚C for 50 sec; 72˚C for 10 mins. The humanized heavy chain's gene has a Hind III restriction enzyme site and a signal sequence at the 5' end and the translation stop codon TAA and an EcoRI restriction enzyme site at the 3' end. The signal sequence was ATGGATTTTCAGGTGCAGATTTTCAGCTTCCTGCTAATCAGTGCCTCAG TCATAATATCCAGAGGA (SEQ ID NO: 26). The PCR product was separated by agarose gel electrophoresis, and the target band was recovered and cloned into pGEMT vector. Positive clones were selected and sequenced. The clones containing the correct sequence were selected and digested with Hind III and EcoR I. The heavy chain fragment of the humanized antibody 1A12 (h1A12VHaCH) was purified and recovered by agarose gel electrophoresis, linked to Hind III and EcoR I double-digested plasmid pcDNA3.1 (+) (Invitrogen, USA) by T4 DNA ligase (Invitrogen) to construct a humanized heavy chain eukaryotic expression vector pcDNA 3.1 (+) (h1A12VHaCH).

The humanized light chain's gene was synthesized by overlap PCR using the h1A12VLa gene and the pGEM-T/CL vector as the templates, wherein the reaction condition was; 95˚C for 15 mins; 30 cycles of 94˚C for 50 sec, 58˚C for 50 sec, 72˚C for 50 sec; 72˚C for 10 mins. The resultant PCR product h1A12VLaCL has a Hind III restriction enzyme site and a signal sequence at the 5' end and a translation stop codon TAA and an EcoRI restriction enzyme site at the 3' end. The signal sequence is ATGGATTTTCAGGTGCAGATTTTCAGCTTCC TGCTAATCAGTGCCTCAGTCATAATATCCAGAGGA (SEQ ID NO: 26). The clones containing the correct sequence were selected and digested with Hind III and EcoR I. The light chain fragment of the humanized antibody 1A12 (h1A12VLaCL) was purified and recovered by agarose gel electrophoresis, linked to Hind III and EcoR I double-digested plasmid pcDNA3.1/ZEO (+) (Invitrogen, USA) by T4 DNA ligase (Invitrogen) to construct a humanized heavy chain eukaryotic expression vector pcDNA3.1/ZEO (+)(h1A12VLaCL).

The constructed expression vectors of the humanized heavy chain and light chain were used to co-transfect the COS-1 cells (ATCC CRL 1650) using the liposome method. 72 hours later, the supernatant was collected, and the content of the humanized antibody (h1A12Ha/h1A12La) therein was determined via ELISA. An ELISA plate was coated with the goat anti-human IgG (Fc) and blocked with 2% BSA-PBS at 37˚C for 2 hours. The supernatant to be tested and the standard (Human myeloma IgG1, k) were added into the wells, incubated at 37˚C for 2 hours. HRP-goat anti-human κ was added to induce the binding reaction at 37˚C for 1 hour. TMB was added and 37˚C incubated for 5 minutes. Finally,

$H_2SO_4$ was added to terminate the reaction, and the value of OD450 was determined.

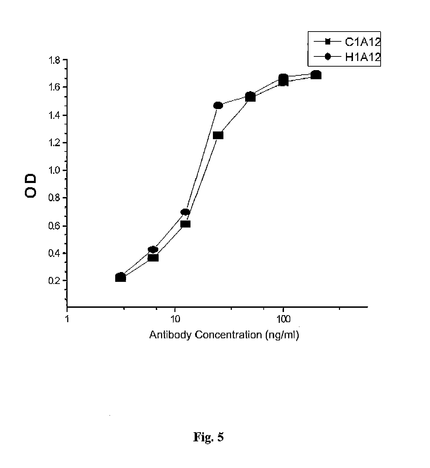

An ELISA plate was coated with 2ug/ml OPN. The binding activity of the humanized antibody obtained from the transfection was detected by ELISA. Results shown that the humanized antibody composed of h1A12Ha and h1A12La (h1A12Ha/h1A12La) lost almost all the activity, in comparison with the chimeric antibody c1A12. This indicated that, in order to obtain a humanized antibody with high-affinity, more analysis was needed to identify the murine residues in the FR region which may be important for the binding activity of h1A12, and reverse mutation might be needed. By analyzing the modeled three-dimensional structure of h1A12's variable region (Figure 3), we found that there are nine residues in the FR regions within a 5Å distance from the CDR that may affect the original conformation of the CDR, and that are different from the residues at the corresponding positions in the human source template. They are L3Leu, L45Lys, L46Leu, H24Ser, H38Lys, H48Ile and H94Ser. These murine amino acid residues were reserved in the constructed CDR-grafted antibody to construct the humanized antibody (h1A12Hb/h1A12Lb). The amino acid sequences of the h1A12Hb's and the h1A12Lb's variable regions were shown in Figure 4. SEQ ID NO: 18 and SEQ ID NO: 19 show the nucleotide sequence and the amino acid sequence of h1A12Hb's variable region respectively. SEQ ID NO: 20 and SEQ ID NO: 21 show the nucleotide sequence and the amino acid sequence of h1A12Lb's variable region respectively. The genes of the humanized antibody's VH and VL (h1A12VHb/h1A12VLb) were synthesized respectively. The expression vector of the light chain, pcDNA3.1/ZEO (+) (h1A12VLbCL), and the expression vector of the heavy chain, pcDNA3.1 (+) (h1A12VHbCH), were constructed in the same method as that used for the humanized antibody (h1A12Ha/h1A12La). Then, expression vectors of the light and the heavy chain were used to co-transfect the COS-1 cells. The antibody's antigen-binding activity was determined by ELISA. Specifically, an ELISA plate was coated with OPN protein (2ug/ml) coated at 4˚C overnight, and was blocked with 2% BSA-PBS for 2h at 37˚C. The supernatant of h1A12 culture to be tested was added into the plate, and the plate was incubated at 37˚C for 2h. HRP-rabbit anti-human IgG was added into the plate for binding reaction (incubated at 37˚C for 1h). TMB was then added and reacted at 37˚C for 5min before adding $H_2SO_4$ to terminate the reaction. The values of A450 and A630 were measured. The result of h1A12's specific binding to OPN was shown in Fig. 5. The binding activity of h1A12 to OPN was similar to that of c1A12, and the humanized antibody (h1A12Hb/h1A12Lb) was named as h1A12.

Example 11. The stable expression and purification of the humanized anti-OPN antibody h1A12

The constructed plasmids of c1A12 or h1A12 heavy chain and light chain were used to co-transfect CHO-K1 cells (ATCC CRL-9618) by liposome method. 24 hours later, the media was changed to a selective media containing 600μg/ml G418 and 250μg/ml Zeocin for the selection of resistant clones. The supernatant of cell culture was screened for high expression clones by ELISA assay. The obtained high expression clones were expanded in a serum-free growth medium. Chimeric antibody c1A12 or humanized antibody h1A12 were isolated and purified by protein A affinity column (GE), and were finally quantified by UV absorption.

**Experiment I. Effects of the anti-hOPN antibodis against tumor cells**

MDA-MB-435s was purchased from the Institute of Cell Biology of Chinese Academy of Sciences (Shanghai, China), and 23C3D3 was used as the irrelevant antibody.

Experiment I-1. Cell adhesion

A 96-well plate (Greiner) was coated with 10μg/ml hOPN (prepared in Example 3) or BSA (SIGMA) at 4˚C overnight, and was then blocked with 1% BSA/PBS at 37˚C for 1 hour to block any non-specific binding sites. The MDA-MB-435s cells were detached using 0.2% EDTA and then re-suspended in 0.25% BSA/DMEM at the concentration of $5 \times 10^5$ cells/ml. To each well, 100 μl ($5\times10^4$) MDA-MB-435s cells were added in the presence of various anti-OPN antibodies (25μg/ml) murine anti-human OPN antibody 1A12, the human-murine Chimeric antibody c1A12 or the humanized antibody h1A12 as treatment groups and the irrelevant antibody 23C3 as control (25μg/ml). After incubation at 37˚C for 2 hrs, the medium was removed and washed twice with PBS gently. The adherent cells were fixed in 1% formaldehyde at 44˚C for 10 minutes, stained with 0.5% crystal violet after washed with PBS and lysed with 2% Triton X-100 (50 μl). The absorbance was measured at 595 nm.

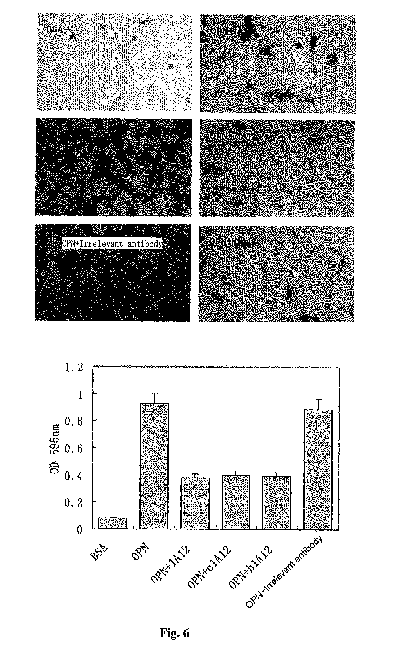

The result was shown in Figure 6, wherein the anti-hOPN antibodies at a level of 25 μg/ml could effectively block the binding between the MDA-MB-435s cells and hOPN, wherein, 1A12, c1A12 and h1A12 did not shown significant differences. The blocking effect was not observed with the irrelevant antibody as control.

Experiment I-2. Cell invasion assay

The assay of cell invasion was carried out using the Transwell system (Coming) with the pore size of 8 mm.

The basement membrane of the upper chamber was coated with the artificial matrix gel (Matrigel). After being dried in a fume hood, the matrigel was hydrated with DMEM at 37°C for I hour. MDA-MB-435s cells were detached using 0.2% EDTA and suspended in 0,25% BSA/DMEM at the concentration of $5 \times 10^5$ cells/ml. 100 μl of the obtained cell suspension as well as the following 25 μg/ml of antibodies were respectively added into the upper chamber of each well: 1A12 (murine anti-human OPN antibody), c1A12 (human-mouse chimeric antibody), h1A12 (humanized antibody), and 23C3 (irrelevant antibody control). To lower chamber, 0.25% of BSA/DMEM (with or without OPN) was added. The system was then incubated in incubator at 37°C for 24 hours. After that, the cells remaining in the upper chamber was wiped away using a cotton swab, and the cells penetrate through the basement membrane into the lower chamber were washed with PBS, fixed with 1% formaldehyde, and then stained with 0.5% crystal violet. The cells were counted in the field at 200× magnification under a microscope.

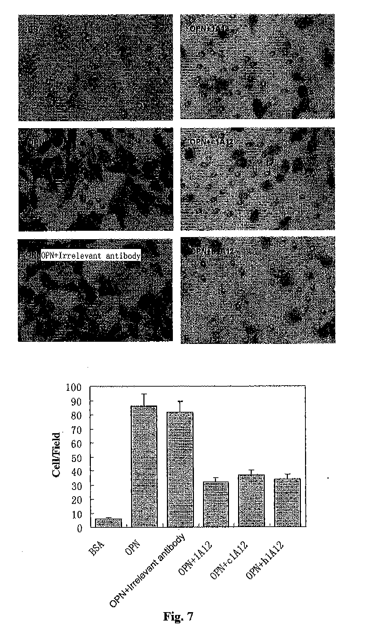

The results, shown in Figure 7, indicated that the anti-hOPN antibodis at the level of 25 μg/ml could effectively block the invasion of the basement membrane by MDA-MB-435s in the presence of hOPN, which was not observed with the irrelevant antibody as control. There were no significant differences among 1A12, c1A12 and h1A12.

Experiment I-3. Scratch wound healing assay

The MDA-MB-435s cells cultured in a 12-wells plate to almost confluent (90%) washed with PBS and starved overnight with serum-free DMEM. Scratches were made on the cell monolayer using a 10 μl pipette, and PBS was added to wash away the floating cells. The test groups were treated with 25 μg/ml of the murine anti-human OPN antibody 1A12, the human-murine chimeric antibody c1A12 and the humanized antibody h1A12, and the control group, 25 μg/ml of the irrelevant antibody. The cells were incubated in an incubator at 37°C for 24 hours. The result was expressed as the number of the cells that cross the base line.

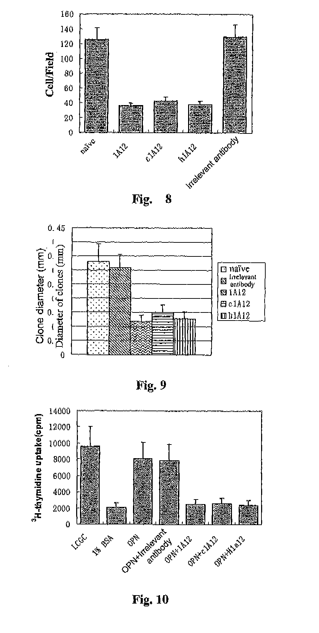

The result was shown in Figure 8. The anti-OPN monoclonal antibodies at the concentration of 25 μg/ml could effectively inhibit the reparation of the scratches and damages, which was not observed with the control. There were no significant differences among 1A12, c1A12 and h1A12.

Experiment I-4. Clone Formation on Soft Agar assay

The assay of clone formation on soft agar was conducted using a double-layer soft agar system. 2.5% melted agar was mixed the DMEM medium pre-warmed at 37°C to formulate a 0.5% agar solution, which was then diluted with DMEM to a 0.3% agar solution. To each well of a 24-well plate was added 500 μl of a 0.5% agar solution, which was allowed to gel at 4°C, and then kept warm in an incubator at 37°C. The MDA-MB-435s cells were detached using 0.2% EDTA and suspended in the 0.3% agar solution at the concentration of $5 \times 10^3$ cells/ml. 500 μl of the cell suspension was added each well and left to gel. From the second day, the cells were respectively treated with 1A12, c1A12, h1A12 or the irrelevant antibody (23C3D3) (25 μg/ml) every other day. After 3 weeks, the colony formation ability was evaluated by measuring the size of colonies containing more than 10 cells.

The result was shown in Figure 9. Anti-OPN antibodies 1A12 could inhibit the clone size formed by the MDA-MB-435s cells on soft agar, which was not observed with the control. There are no significant differences among 1A12, c1A12 and h1A12. These results indicate that the antibodies could effectively inhibit the formation of metastatic foci, and that the chimeric antibody and the humanized antibody maintained the biological function of murine-originated monoclonal antibody.

**Experiment II. The inhibitory effect of the anti-OPN antibodies on angiogenesis**

Experiment II-1. Determination of the proliferation of the human vascular endothelial cell (HUVEC) by $^3$H-TdR incorporation

HUVEC cell proliferation was measured by the $^3$H-TdR incorporation method to determine the protective effect of OPN on cell survival and the inhibitory effect of the anti-OPN antibodies on the OPN protection. Each well of a 96-well plate was inoculated with $2 \times 10^4$ HUVECs and cultured in a complete medium for 24 hours. The cells were then treated as following for 24 hours:

    i. M200 + LCGS (supplement culture)
    ii. M200 + 1% BSA
    iii. M200 + 1% BSA + OPN
    iv. M200 + 1% BSA + OPN + murine anti-OPN antibody 1A12
    v. M200 + 1% BSA + OPN + human-murine chimeric anti-OPN antibody c1A12
    vi. M200 + 1% BSA + OPN + humanized anti-OPN antibody h1A12

vii. M200 + 1% BSA + OPN + irrelevant antibody

24 hours later, 1 uCi/well $^3$H-thymidine was added and, after being incubated at 37°C for 6 hours, washed once with PBS. The plate was treated with ice-cold 10% trichloroacetic acid/H$_2$O at 4°C for 10 min, and then washed for two times with, ice-cold ddH$_2$O. Cytolysis was conducted by adding into each well 0.1 ml of 0.5 N NaOH, 0.5% SDS at room temperature for 10 min. After adding 0.2 ml of 0.5 N HCl and mixing, the cells were collected using a multiple cell collector.
The result of $^3$H-TdR incorporation was shown in Figure 10. The result showed that, in the presence of OPN, the $^3$H-TdR incorporation was 3.08 ± 0.64 times higher than that in the 1% BSA treatment group, while with the addition of the anti-OPN antibody 1A12, c1A12 or h1A12, the $^3$H-TdR incorporation was significantly lower than the OPN group. The results indicated that, OPN enhanced the survival of HUVECs in the absence of serum, which could, however, be significantly inhibited by the anti-OPN antibodies (P <0.01).

Experiment II-2. The inhibitory effect of 1A12, c1A12 and h1A12 on the formation of the capillary-like structure

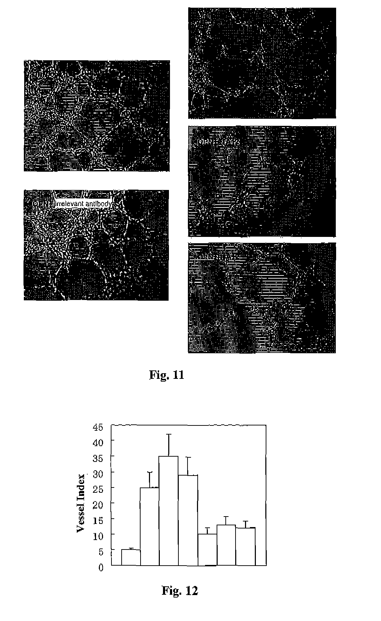

HUVECs were cultured in the M200 medium containing LCGS to 80% confluence. The matrigel stored at -20°C was took out the day before experiment and naturally thawed at 4°C for 3 to 12 hours. To each well of a 96-well plate was added 60 μl matrigel using a pre-cooled pipet with carefully removing the bubbles, wherein the whole procedure was carried on ice. The loaded plate was incubated at 37°C for 1 hour to gel the matrigel. To each well, 100 μl HUVECs (2 × 10$^4$/well) was gently added onto the surface of matrigel. To the treatment groups were further added the anti-hOPN antibodies 1A12, c1A12 and h1A12. The plate was incubated at 37°C for 12 hours, and the formation of the capillary-like structure was observed 6 hours after.
The formation of the capillary-like structure in HUVECs was shown in Figure 11. The result showed that the anti-hOPN antibodies could reduce the potency of forming the capillary-like structure in HUVECs, which was not observed with the control. There were no significant differences among 1A12, c1A12 and h1A12.

Experiment II-3. 1A12, c1A12 and h1A12 inhibit the angiogenesis in chicken embryo chorioallantoic membrane (CAM)

The Leghorn chicken embryos were purchased from the farm with a fertilization rate of >90%. The 8-day old chicken embryos were selected. After sterilization, a small hole was drilled in the center of the top air chamber of the embryo with a lancet, the surrounding shell with the inner membrane was removed with caution to create an opening of 1 cm × 1 cm. Then the following analytes/gelatin sponge were added to the CAM surface: i) PBS; ii) HOPN; iii) human OPN (250 ng); iv) human OPN (250 ng) + the irrelevant antibody as control (1 μg); v) human OPN (250 ng) + the murine anti-OPN antibody 1A12 (1 μg); vi) human OPN (250 ng) + the murine-human chimeric anti-OPN antibody c1A12 (1 μg); vii) human OPN ( 250 ng) + the humanized anti-OPN antibody h1A12 (1 μg),
**[0115]** The analytes/ gelatin sponge were added to the CAM surface, which was far away from the already-formed dense vascular network. The opening was sealed with a sterilized transparent adhesive tape, and the embryos were incubated for additional 72 hours or even longer. The number of vessels within an area edging 2 mm from the edges of the carrier slide was counted under a dissecting microscope to study the angiogenesis using the "vascular index" (Ribatti, D., B, Nico, A, Vacca et al., The gelatin sponge-chorioallantoic membrane assay. Nat Protoc, 2006, 1 (1): 85-91). The number of vessel was counted at the same magnification under the dissecting microscope. The result in each sample group was expressed as "mean ± standard deviation (x ± S)". The difference among the groups was compared by multiple comparison variance q-test using the SPSS statistical software.
The result was shown in Figure 12, wherein the human OPN protein could significantly enhance the angiogenesis in the chicken embryo's CAM, in comparison with PBS. The microvessels were radially extended from the gelatin sponge as the center. The difference was statistically significant (P <0.01). The capacity of OPN to promote angiogenesis was similar to that of HOPN. The anti-OPN antibodies 1A12 and 2H8 could significantly inhibit the OPN-induced angiogenesis in CAM (P <0.01).

Experiment II-4. 1A12, c1A12 and h1A12 inhibit neovascularization in rabbit corneal

The Hydron powder was dissolved in anhydrous ethanol under sterile condition to the final concentration of 12% (w/v), and dissolved with agitation under 37°C to formulate the 12% Hydron cast solution, which was then stored at room temperature, A quota of sucralfate powder formulated in sterile Milli Q water under sterile conditions as a suspension with the final concentration of 100 μg/μl, which was stored at 4°C, and vortexed to homogeneous intermediately before use. Sustained-release pellets were prepared to each containing 200 ng hOPN and 50 μg sucralfate and, for the antibody groups, 1 μg anti-OPN antibodies, and for the control, the irrelevant antibody. The 12% Hydron cast solution and the hOPN-sucralfate-PBS blend were mixed as the ratio of 1:1 (volume ratio), vortexed for 1 min. Then 5 μl mixed Hydron-sucralfate-hOPN solution was added onto a sterilized Parafilm surface, dried for about 30 min on a super-clean bench for a complete polymerization. The polymers were then shaped into uniform circular granules with a 2 mm diameter using an eye tweezers. The granules were stored at -20˚C. Animals were grouped in the same way as in Experiment II-3, each group consisting of four eyes.

The rabbit was anesthetized at ear edge vein with 3% sodium pentobarbital (1 ml/kg body weight) and on both corneal surfaces with 1% tetracaine, The thickness of the cornea was measured using an ultrasonic cornea thickness gauge. A 3 mm half-depth incision was made at the center of the cornea using an ophthalmic knife, and an undermining tunnel toward the 12 o'clock or 3 o'clock position on the corneoscleral margin was made using a 2 mm-wide scleral tunnel knife, whereby the tunnel lied within the corneal layer with the top of the tunnel about 1 mm distant from the limbus corneae. The Hydron/sucralfate sustained-release pellet was implanted into the top of tunnel using micro-tweezers. The cuts were treated with chlortetracycline eye ointment after the surgery. From Day 1 after the surgery, the double-blinded observer observed the rabbit corneal neovascularization under the slit-lamp each day, until the Day 10. The length (VL) and the vascular clock angle (CN, 30˚ = 1CN) of the longest vessel were measured, and the area of neovascularization was calculated according to the following formula:

$$\text{Area (mm}^2) = 0.2 \times \pi \times \text{VL (mm)} \times \text{CN (mm)}$$

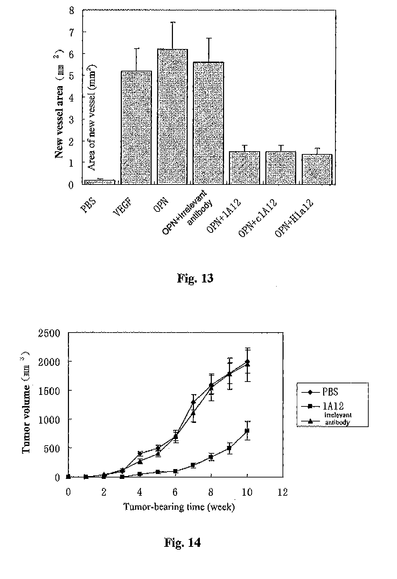

The result was shown in Figure 13. In the 1A12-, c1A12- and h1A12- treated groups, the neovascularization was significantly reduced relative to the OPN group, with the length of the newly generated blood vessels significantly reduced and area was significantly shrunk relative to the irrelevant antibody group (P <0.01). There was no significant difference among the 1A12, the c1A12 and the h1A12 groups.

**Experiment III. Murine anti-human OPN antibody 1A12 inhibits tumorous metastasis and angiogenesis in mice**

Experiment III-1, Murine anti-human OPN antibody 1A12 inhibits tumor metastasis in mice

The MDA-MB-435s cells were detached and re-suspended in serum-free DMEM to the concentration of $5 \times 10^7$ cells/ml. Each group of 10 female 4 to 6-week old nude mice were anaesthetized by injection of sodium pentobarbital (ip.). Under sterile condition, the skin was sterilized, and a small incision (0.5-mm) was made in the skin over the lateral thorax below the right limb. The mammary fat pads (mfp) were exposed and MDA-MB-435s cells ($5 \times 10^6$ in 100 μl DMEM) were orthotopically injected into the mfps. The cut was then closed. The animals were randomly divided into four groups, and each accepted the treatment as specified below from the day after the tumor cell inoculation:

- 1A12: 5 mg/kg, 2 times per week;
- Control antibody: 5 mg/kg, 2 times per week.

The tumor formation in nude mice was observed once a week, the maxima of the diameter (length) and a perpendicular dimension of the tumor (width) were measured using a caliper, and the volume was calculated as follows: Volume = $0.52 \times$ length $\times$ width$^2$. The changed tumor volumes were plotted versus the time into a tumor growth curve. For 6 of the 12 animals in each treatment group, size of the tumors form in situ were observed for until the 10th week, and for the other 6 animals, spontaneous metastasis into the lung was observed until the primary tumor reached 1000 mm$^3$ in size.

The mice were sacrificed by cervical dislocation at the end of the 10th week after inoculation. For each group, tumor tissues were isolated, fixed with 10% neutral formalin and embedded in paraffin, and the tumor tissue and lung tissue were stained with H & E for analysis. The size and number of the metastasis in lung were observed under microscope.

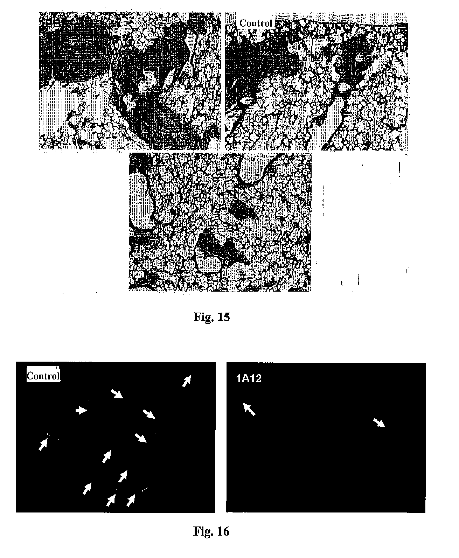

The tumor size was plotted versus time into a tumor growth curve. The result was shown in Figure 14, wherein, by the 10th week, the tumor in the group treated with an anti-OPN antibody (1A12) was significantly smaller than those in the irrelevant antibody treated group (P <0.05). As shown by the histological sections of the metastatic foci in lung of the nude mice in Figure 15, not only the number but also the size of the metastasis was reduced after the treatment with the anti-OPN antibody 1A12. In the contrast, big foci, with some even fusing into bigger metastasis, were generally observed in the lung tissue of the control group.

Experiment III-2. The effect of the murine anti-human OPN antibody 1A12 on the microvessel density (MVD) in tumor

The tissue of the in situ tumor in mouse was isolated, and speed-frozen in liquid nitrogen. 8 um serial sections were made in a constant cold box frozen section machine, and dried for 5 min, before being fixed with acetone at -20˚C for 30 min, and then stained with IHC. Incubation with the rat anti-mouse monoclonal antibody CD31 (working concentration 1:100) was carried out at 4°C overnight. The secondary antibody was goat anti-rat IgG/PE (working concentration 1:100). After Hochest33258 contrast stain, photographs were taken under the fluorescence microscope.

The CD31 protein was mainly expressed on the membrane of the vascular endothelial cells, and was expressed in individual vascular endothelial cells and microvessel. Intra-tumor microvessel could be visualized with an anti CD31 antibody (Figure 16). The microscopic result showed that, MVD was significantly less in the anti OPN antibody 1A12 treatment group than that in the irrelevant control (P <0.01).

Experiment IV. Identification of monoclonal antibody 1A12's epitope

Experiment IV-1. Epitope mapping of murine anti-hOPN monoclonal antibody 1A12

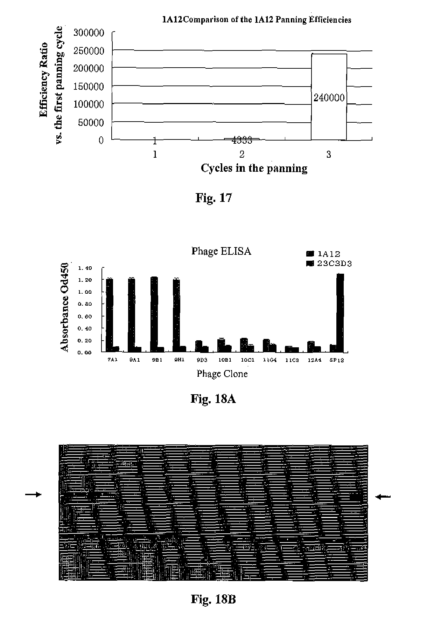

Biopanning of random peptide library was used to map the epitope of 1A12. The whole process was carried out on a 96-well plate. The plate was coated with antibody 1A12 (100ug/ml, 100ul/well) at 4°C overnight, 10% skimmed milk (diluted with TBST) blocked overnight, and 1 × TBST (Tween-20, 0.1%) washed for six times. Then, to the plate were added the phage display random peptide library (purchased from NEB, Ph.D.-12™ Phage Display Peptide Library Kit) $4 \times 10^{10}$ pfu and 100 $\mu$l normal mouse serum and gently shaken at room temperature for 1 hour. The plate was washed with 1 × TBST (Tween-20, 0.1%) for 15 times, and was then eluted with 1mg/ml BSA in glycine-Cl (pH 2.2) with gently shaking at room temperature for 15 minutes, before being finally neutralized with 15ul Tris-Cl (pH 9.1). 10ul of the eluate was used for titering, and the rest for amplification. After precipitated by PEG/NaCl, the product was determined for its titration, and was subject to the second round of panning. The third round of panning follows the same process, For each round, phages with the same number ($4 \times 10^{10}$ pfu) were added, and the output of the binding phage was $6.9 \times 10^2$ pfu, $2.99 \times 10^6$ pfu and $1.69 \times 10^8$ pfu, respectively. The panning efficiency increased at a 4333- and a 240000-fold relative to that of the first round. The result suggested that, the effect of enrichment by panning is significant, see Figure 17.

Experiment IV-2. Epitope mapping of irrelevant control antibody 23C3D3

Biopanning of random peptide library was used to map the epitope. The whole the process was carried out on a 96-well plate. The plate was coated with antibody 23C3D3 (100ug/ml, 100ul/well) at 4°C overnight, 10% skimmed milk (diluted with TBST) blocked overnight, and 1 × TBST (Tween-20, 0.1%) washed for six times. Then, to the plate were added the phage random peptide library (purchased from NEB, Ph.D.-12™ Phage Display Peptide Library Kit) $4 \times 10^{10}$ pfu and 100 $\mu$l normal mouse serum, and gently shaken at room temperature for 1 hour. The plate was washed with 1 × TBST (Tween-20, 0.1%) for 15 times, and eluted with 1mg/ml BSA in glycine-Cl (pH 2.2) with gently shaking at room temperature for 15 minutes, before finally being neutralized with 15ul Tris-Cl (pH 9.1). 10ul of the eluate was used for titering and the rest for amplification. After precipitated by PEG/NaCl, the amplification product was determined for its titration, and was subject to the second round of panning. The third round of panning follows the same process. The plate was coated with antibody 23C3D3 and examined by ELISA. A positive clone, 5F12, was selected and used as the control phage.

Experiment IV-3. Phage clone ELISA and Western blot assay

ELISA was carried on a 96-well plate, wherein each well was coated with 50ul of 100ug/ml 1A12 monoclonal antibody at 4°C overnight, blocked with 10% skimmed milk (TBST diluted) at 37°C for 2 hours, and washed with 1 × TBST (Tween-20, 0.1%) for five times. The supernatant of amplification of each monoclonal phage was diluted with 1 × TBS to $5 \times 10^8$ pfu/50ul. The control antibody is murine anti-human OPN monoclonal antibody (Santa Cruz), and the negative control is 5F12 (23C3D3 positive clone). The Binding reaction was performed at room temperature for 1 hour. After being washed with 1 × TBST (Tween-20, 0.1%) for five times, each well was added with 200ul 1:5000 diluted HRP labeled anti-M13 antibody (Pharmacia # 27-9411-01), shaken for 1 hour at room temperature for the reaction. After being washed with 1 × TBST (Tween-20, 0.1%) for five times, the fresh substrate of the ELISA detection kit (A:B = 1: 1, 50ul/well) (Jinmei Biotech. Co. Shanghai) was added and incubated at room temperature for 1-5 minutes, before 2N $H_2SO_4$ was added to terminate the reaction. Each clone was tested for 1A12 and control antibody 23C3D3 in triplicate. OD450 data showed that the positive clones were specific for the antibody, see Figure 18A.

Western blot process: the supernatant of the amplification of each phage's monoclone was purified by 20% PEG/NaCl precipitation, and then subject to 10% SDS-PAGE electrophoresis ($1 \times 10^{10}$ pfu/lane, 360mA, 1 hour). The result was transferred to a nitrocellulose membrane, blocked with 10% skim milk at 4°C overnight or at room temperature for two hours. After being washed with 1 × TBST (Tween-20, 0.1%) for three times, the membrane was treated with 10ug/ml primary antibody at room temperature for 1 hour. Then, the membrane was washed with 1 × TBST (Tween-20, 0.1%) for five times (10 min/each), treated with 1:1000 diluted HRP labeled rabbit anti-mouse IgG (Beijing Zhongshan Company) at room temperature for 1 hour. After being treated with the ECL kit (Tiangen Company) for 1-2 minutes, medical blue-sensitive X-ray film was exposed. In figure 18B, on the left was shown the hybridization result with antibody 1A12, on the right, was shown the hybridization result of the irrelevant antibody 23C3D3, the arrows indicating the bands of target. These results indicated that, the positive clones were specific to the antibody, see Figure 18B.

Experiment IV-4. Sequencing and sequence analysis of the epitope recognized by the antibody

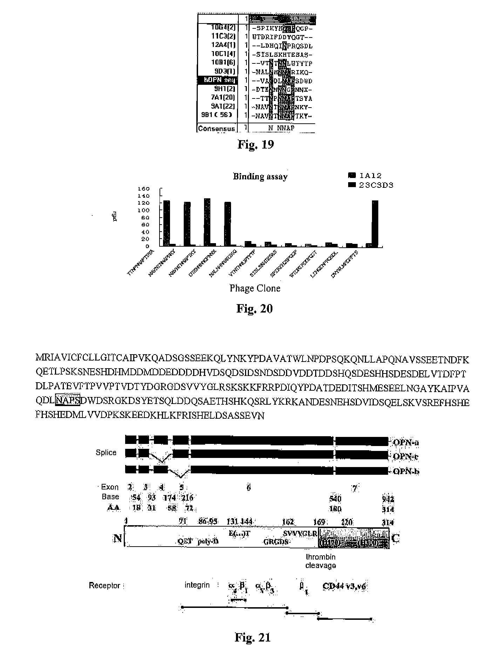

The Single-stranded DNA Extraction Kit (Shanghai Jierui Company) was used to prepare the template, sequencing using the -96 primers, reading the sequence with Chromas software. 4 independent sequences were obtained among 100 positive clones. After analyzed with AlignX software, we got a consensus sequence of NXNNAP. Further, since G and A are both non-polar aromatic amino acid, while S, T, N and P are all uncharged polar amino acid, a homologous sequence of NAPS can be found in the sequence of the antigen hOPN. Therefore, the putative epitope specific for 1A12 was supposed to be NAPS. The results were shown in Figure 19.

Experiment IV-5. Binding between the Phage clones and the antibody

Phage clones were added at $5 \times 10^7$ pfu to an antibody-coated 96 well plate, subject to the panning under the same conditions. (Control: antibody 23C3D3, and irrelevant control: phage 5F12). The eluate of the phage was titered (according to Blood 2006-04-014639). The results showed that in the epitope NAPS in hOPN, APS played an important role in mediating the binding between 1A12 and hOPN, whereas N or NN alone could not mediate the binding. The A at position 2 of the epitope motif could be substituted by G without affecting the binding capacity, both being non-polar aliphatic amino acid. The amino acid at position 4 of the epitope motif would not affect the binding capacity as long as it was an uncharged polar amino acid (eg., S, T, N, P). The result of this experiment shows that the 1A12's epitope was NAPS, see Figure 20.

The results showed that, the epitope specifically recognized by antibody1A12 was NAPS, which was located in the seventh exon of OPN, and was a newly found epitope. The location and sequence of the epitope were shown in Figure 21, and the sequence was also set forth in SEQ ID NO: 7.

All documents mentioned in this specification are herein incorporated by reference to the same extent as if each individual document was specifically and individually indicated to be incorporated by reference. Further, it is understood that, after reading this specification, those skilled in the art can make variations and modifications to the present invention, and the scope of protection of the invention is determined in particular by the following claims.

## Claims

1.  A functional epitope of osteopontin, wherein the functional epitope is NXPY, and wherein X= A or G, and Y= S, T, N or P.

2.  An anti-osteopontin monoclonal antibody, which specifically binds to the functional epitope of claim 1.

3.  The monoclonal antibody of claim 2, wherein the amino acid sequence of the CDR in the heavy chain variable region of the monoclonal antibody is selected from the group consisting of: GYTFTTYVMH, YTNPYNDGSKYNEKFKG and HYGGSPAY; and the amino acid sequence of the CDR in the light chain variable region is selected from the group consisting of: RSSQSLVHSNGNTYLH, KVSNRFS and SQSTHVPWT.

4.  The monoclonal antibody of claim 2, wherein the amino acid sequence of the heavy chain variable region in the monoclonal antibody is SEQ ID NO: 4 or SEQ ID NO: 19, and the amino acid sequence the light chain variable region in the monoclonal antibody is SEQ ID NO: 6 or SEQ ID NO: 21.

5.  The monoclonal antibody of claim 2, wherein the constant region of the monoclonal antibody is a mouse antibody constant region or a human antibody constant region.

6.  A DNA molecule, which encodes the monoclonal antibody of any of claims 2-5.

7.  A vector, which contains the DNA molecule of claim 6.

8.  A host cell, which contains the vector of claim 7, or contains the DNA molecule of claim 6 integrated in its genome.

9.  An immunoconjugate, which contains:

    (a) the monoclonal antibody of claim 2; and
    (b) a conjugating moiety selected from the group consisting of a drug, a toxin, a cytokine, a radionuclide, and an enzyme.

10. Use of the anti-osteopontin monoclonal antibody of claim 2 or the immunoconjugate of claim 9 in the manufacture of antitumor agents.

11. A pharmaceutical composition comprising the anti-osteopontin monoclonal antibody of claim 2 or the immunoconjugate of claim 9, and pharmaceutically acceptable carriers.

12. A kit for the detection of osteopontin, which contains the anti-osteopontin monoclonal antibody of claim 2 or the immunoconjugate of claim 9.

**13.** A method for detecting the presence of or determining the content of osteopontin in a biological sample, which comprises the following steps:

(i) contacting the sample with the anti-osteopontin monoclonal antibody of claim 2 or the immunoconjugate of claim 9;
(ii) detecting the formation of an antigen-antibody complex,

wherein the formation of the antigen-antibody complex suggests the presence of osteopontin in the sample, or wherein the quantitatively determined content of the antigen-antibody complex reflects the content of osteopontin in the sample.

M    hOPN mOPN

97KDa ‾‾‾‾

66KDa ‾‾‾‾

45KDa ‾‾‾‾

30KDa ‾‾‾‾

**Fig.1**

1A12                Commercially available anti-OPN antibody
   hOPN      mOPN              hOPN      mOPN

**Fig. 2**

**Fig. 3**

**A**

```
            1         10        20        30        40        50
1A12VH    QVQLEQSGPELVKPGASVKMSCKSS[GYTFTTYVMH]WVKQKPGQGLEWIG[Y
CAA79298  QVQLVQSGAEVKKPGASVKVSCKAS[GYTFTGYYMH]WVRQAPGQGLEWMG[W
H1A12VHa  -------------------------[-----T-V--]---------------[Y
H1A12VHb  -----------------S-[-----T-V--]--K---------I-[Y
            a         60        70        80  a b c     90
1A12VH    INPYNDGSKYNEKFKG]KATLTSDKSSNTAYMELSSLTSEDSAVYYCAS[HY
CAA79298  INPNSGGTNYAQKFQG]RVTITRDTSASTAYMELSSLRSEDTAVYYCAR[DF
H1A12VHa  ---YND-SK-NE--K-]----------------------------------[HY
H1A12VHb  ---YND-SK-NE--K-]-----S-K-------------------------S[HY


              100        110
1A12VH      GGSPAY]WGQGTLVTVSA
CAA79298  FLSGYLDY]WGQGTLVTVSS
H1A12VHa    G-SPA-]------------
H1A12VHb    G-SPA-]------------
```

**B**

```
            1         10        20        30 a b c d e   40
1A12VL    DILMTQTPLSLPVSLGDQASISC[RSSQSLVHSNGNTYLH]WYLQKPGQSP
BAC01734  DVVMTQSPLSLPVTLGQPASISC[RSSQSLVHSDGNTYLN]WFQQRPGQSP
H1A12VLa  ----------------------[---------N-----H]----------
H1A12VLb  --L-------------------[---------N-----H]----------


              50        60        70        80        90
1A12VL    KLLIY[KVSNRFS]GVPDRFSGSGSGTDFTLKISRVEAEDLGVYFC[SQSTHV
BAC01734  RRLIY[KVSNRDS]GVPDRFSGSGSGTDFTLKISRVEAEDVGVYYC[MQGTHW
H1A12VLa  -----[-----F-]--------------------------------[S-S--V
H1A12VLb  KL---[-----F-]--------------------------------[S-S--V


              100
1A12VL    PWT]FGGGTKLEIKR
BAC01734  PLT]FGGGTKVEIKR
H1A12VLa  -W-]-----------
H1A12VLb  -W-]-----------
```

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

1A12Comparison of the 1A12 Panning Efficiencies

**Fig. 17**

Phage ELISA

**Fig. 18A**

**Fig. 18B**

Fig. 19

Phage Clone

Fig. 20

MRIAVICFCLLGITCAIPVKQADSGSSEEKQLYNKYPDAVATWLNPDPSQKQNLLAPQNAVSSEETNDFK
QETLPSKSNESHDHMDDMDDEDDDDHVDSQDSIDSNDSDDVDDTDDSHQSDESHHSDESDELVTDFPT
DLPATEVFTPVVPTVDTYDGRGDSVVYGLRSKSKKFRRPDIQYPDATDEDITSHMESEELNGAYKAIPVA
QDLNAPSDWDSRGKDSYETSQLDDQSAETHSHKQSRLYKRKANDESNEHSDVIDSQELSKVSREFHSHE
FHSHEDMLVVDPKSKEEDKHLKFRISHELDSASSEVN

Fig. 21

49

## INTERNATIONAL SEARCH REPORT

International application No.

PCT/CN2008/070576

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)

IPC: C07K; C12N; A61K; A61P; G01N

Documentation searched other than minimum documentation to the extent that such documents are included in the fields searched

Electronic data base consulted during the international search (name of data base and, where practicable, search terms used)

CPRS; CNKI; WPI; EPODOC; PAJ; MEDLINE and keywords: osteopontin, OPN, epitope, antibody etc.
EMBL; GenBank: the amino acid sequences of functional epitopes of the osteopontin, SEQ ID NOs: 4, 6, 19 and 21.

**C. DOCUMENTS CONSIDERED TO BE RELEVANT**

| Category* | Citation of document, with indication, where appropriate, of the relevant passages | Relevant to claim No. |
|---|---|---|
| PX<br><br>PA | CN 101066999 A (SHANGHAI ZHONGXIN GUOJIAN PHARM IND CO)<br>07 Nov. 2007(07.11.2007)<br>claims 1-7, examples 5 and 6 of the description, SEQ ID NOs: 1 and 2<br>the whole document | 2-3, 5-9, 12-13<br>1, 4, 6-8, 10-11 |

☒ Further documents are listed in the continuation of Box C.　　☒ See patent family annex.

| | |
|---|---|
| *　　Special categories of cited documents: | "T"　later document published after the international filing date or priority date and not in conflict with the application but cited to understand the principle or theory underlying the invention |
| "A"　document defining the general state of the art which is not considered to be of particular relevance | |
| "E"　earlier application or patent but published on or after the international filing date | "X"　document of particular relevance; the claimed invention cannot be considered novel or cannot be considered to involve an inventive step when the document is taken alone |
| "L"　document which may throw doubts on priority claim (S) or which is cited to establish the publication date of another citation or other special reason (as specified) | "Y"　document of particular relevance; the claimed invention cannot be considered to involve an inventive step when the document is combined with one or more other such documents, such combination being obvious to a person skilled in the art |
| "O"　document referring to an oral disclosure, use, exhibition or other means | |
| "P"　document published prior to the international filing date but later than the priority date claimed | "&"document member of the same patent family |

| Date of the actual completion of the international search | Date of mailing of the international search report |
|---|---|
| 23 Jun. 2008(23.06.2008) | **03 Jul. 2008 (03.07.2008)** |

| Name and mailing address of the ISA/CN<br>The State Intellectual Property Office, the P.R.China<br>6 Xitucheng Rd., Jimen Bridge, Haidian District, Beijing, China<br>100088<br>Facsimile No. 86-10-62019451 | Authorized officer<br><br>LIN, Junkai<br><br>Telephone No. (86-10)62411095 |

Form PCT/ISA/210 (second sheet) (April 2007)

| INTERNATIONAL SEARCH REPORT | International application No. |
|---|---|
| | PCT/CN2008/070576 |

C (Continuation).    DOCUMENTS CONSIDERED TO BE RELEVANT

| Category* | Citation of document, with indication, where appropriate, of the relevant passages | Relevant to claim No. |
|---|---|---|
| A | CN 1513000 A (IMMUNO BIOLOGICAL LAB CO LTD) 14 Jul. 2004(14.07.2004) <br> the whole document | 1-13 |
| A | WO 2006043954 A1 (GENENTECH INC) 27 Apr. 2006(27.04.2006) <br> the whole document | 1-13 |
| A | HOTTA, H. ET AL Detection of Various Epitopes of Murine Osteopontin by Monoclonal Antibodies. Biochem. Biophys. Res. Commun. 02 Apr. 1999(02.04.1999), vol. 257, No. 1, pages 6-11, ISSN 0006-291X <br> the whole document | 1-13 |

Form PCT/ISA/210 (continuation of second sheet ) (April 2007)

## INTERNATIONAL SEARCH REPORT
Information on patent family members

| | International application No. |
|---|---|
| | PCT/CN2008/070576 |

| Patent Documents referred in the Report | Publication Date | Patent Family | Publication Date |
|---|---|---|---|
| CN 101066999 A | 07.11.2007 | None | |
| CN 1513000 A | 14.07.2004 | WO 02081522 A1 | 17.10.2002 |
| | | NO 20034405 A | 05.12.2003 |
| | | EP 1375518 A1 | 02.01.2004 |
| | | BRPI 0208809 A | 09.03.2004 |
| | | KR 20030092022 A | 03.12.2003 |
| | | CZ 20032697 A3 | 14.01.2004 |
| | | AU 2002244968 A1 | 21.10.2002 |
| | | JP 2002579907 T | 29.07.2004 |
| | | KR 20040048899 A | 10.06.2004 |
| | | US 2004234524 A1 | 25.11.2004 |
| | | HU 0401561 A2 | 29.11.2004 |
| | | MXPA 03009052 A | 01.12.2004 |
| | | INKOLNP 200301216 E | 30.09.2005 |
| | | INKOLNP 200400359 E | 17.02.2006 |
| | | CN 1688604 A | 26.10.2005 |
| | | EP 1754719 A2 | 21.02.2007 |
| | | JP 2007106767 A | 26.04.2007 |
| | | RU 2299888 C2 | 27.05.2007 |
| | | INKOL 200601203 A | 20.07.2007 |
| | | AU 2002244968 B2 | 26.07.2007 |
| | | US 2008069815 A1 | 20.03.2008 |
| | | NZ 528483 A | 28.03.2008 |
| WO 2006043954 A1 | 27.04.2006 | EP 1805220 A1 | 11.07.2007 |
| | | AU 2004324192 A1 | 27.04.2006 |
| | | AU 2004324192 A2 | 27.04.2006 |

Form PCT/ISA/210 (patent family annex) (April 2007)

52

## INTERNATIONAL SEARCH REPORT

| International application No. |
| --- |
| PCT/CN2008/070576 |

**CLASSIFICATION OF SUBJECT MATTER**

C07K 5/113 (2006.01) i

C07K 16/18 (2006.01) i

C07K 16/46 (2006.01) i

C12N 15/13 (2006.01) i

C12N 15/63 (2006.01) i

A61K 39/395 (2006.01) i

A61P 35/00 (2006.01) i

G01N 33/577 (2006.01) i

Form PCT/ISA/210 (extra sheet) (April 2007)

## REFERENCES CITED IN THE DESCRIPTION

**Patent documents cited in the description**

- US 4816567 A

**Non-patent literature cited in the description**

- **Yamaguchi H ; Wyckoff J ; Condeelis J.** Cell migration in tumors. *Curr Opin Cell Biol.,* 2005, vol. 17 (5), 559-64
- **Huber MA ; Kraut N ; Beug H.** Molecular requirements for epithelial-mesenchymal transition during tumor progression. *Curr Opin Cell Biol.,* 2005, vol. 17 (5), 548-58
- **Rangaswami H ; Bulbule A ; Kundu GC.** Osteopontin; role in cell signaling and cancer progression. *Trends Cell Biol.,* 2006, vol. 16 (2), 79-87
- **Weber GF ; Ashkar S ; Glimcher MJ ; Cantor H.** Receptor-ligand interaction between CD44 and osteopontin (Eta-1). *Science,* 1996, vol. 271, 509-12
- **Miyauchi A ; Alvarez J ; Greenfield EM et al.** Recognition of osteopontin and related peptides by an $\alpha v \beta 3$ integrin stimulates immediate cell signals in osteoclasts. *J Biol Chem,* 1991, vol. 266, 20369-7
- **Teramoto H ; Castellone MD ; Malek RL ; Letwin N ; Frank B ; Gutkind JS ; Lee NH.** Autocrine activation of an osteopontin-CD44-Rac pathway enhances invasion and transformation by H-RasV12. *Oncogene,* 2005, vol. 24 (3), 489-501
- **Teramoto H et al.** *Oncogene,* 13 January 2005, vol. 24 (3), 489-501
- **Wolf K ; Mazo I ; Leung H et al.** Compensation mechanism in tumor cell migration: mesenchymal-amoeboid transition after blocking of pericellular proteolysis. *J Cell Biol,* 2003, vol. 160, 267-77
- **Nicholson, KM ; Anderson, NG.** The protein kinase B/Akt signaling pathway in human malignancy. *Cell Signal.,* 2002, vol. 14, 381-395
- *J. Biol. Chem.,* 2001, vol. 276, 46024-46030
- **Philip, S. ; Kundu, GC.** Osteopontin induces nuclear factor $\kappa$B mediated promatrix metalloproteinase-2 activation through I kappa B alpha/IKK signaling pathways and curcumin (diferulolylmethane) downregulates these pathways. *J. Biol. Chem.,* 2003, vol. 278, 14487-14497

- **Rangaswami, H. et al.** Nuclear factor inducing kinase plays a crucial role in osteopontin induced MAPK / I$\kappa$Ba kinase dependent nuclear factor-$\kappa$B mediated promatrix metalloproteinase-9 activation. *J. Biol. Chem.,* 2004, vol. 279, 38921-38935
- **Rangaswami, H et al.** JNKI differentially regulates osteopontin induced nuclear factor inducing kinase/MEKK1 dependent activating protein-1-mediated promatrix metalloproteinase-9 activation. *J. Biol. Chem.,* 2005, vol. 280, 19381-19392
- **Goutam Chakraborty et al.** Osteopontin Promotes Vascular Endothelial Growth Factor-Dependent Breast Tumor Growth and Angiogenesis via Autocrine and Paracrine Mechanisms. *Cancer Res,* 2008, vol. 68, 152-161
- **E.A. Kabat ; T.T. Wu ; H.M. Perry ; K.S. Gottesman ; C. Foeller.** Sequences of Proteins of Immunological Interest. United States Department of Health and Human Services, 1991
- **Kohler et al.** *Nature,* 1975, vol. 256, 495
- **Clackson et al.** *Nature,* 1991, vol. 352, 624-628
- **Marks et al.** *J. Mol. Biol.,* 1991, vol. 222, 581-597
- Tissue Culture. Academic Press, 1973
- **Munson et al.** *Anal. Biochem.,* 1980, vol. 107, 220
- Remington's Pharmaceutical Sciences. Mack Pub. Co, 1991
- Molecular Cloning: A Laboratory Manual. Cold spring Harbor Laboratory Press, 1989
- *Cell,* 1980, vol. 22, 197-207
- *Nucleic Acids Research,* 1982, vol. 10, 4071-4079
- **Ribatti, D. ; B, Nico ; A, Vacca et al.** The gelatin sponge-chorioallantoic membrane assay. *Nat Protoc,* 2006, vol. 1 (1), 85-91